# EUROPEAN PATENT APPLICATION

(11) **EP 1 671 647 A1**
(43) Date of publication of application: **21.06.2006**
(21) Application number: 04773540.2
(22) Date of filing: 24.09.2004
(51) Int. Cl.: A61K 45/00, A61K 39/395, A61K 31/351, A61K 45/06, A61P 35/00, C07K 16/22, C12P 21/08

(54) **MEDICAMENT FOR TREATING CANCER**

(30) Priority: 24.09.2003 JP 2003332346
(71) Applicant: KYOWA HAKKO KOGYO CO., LTD., Chiyoda-ku, Tokyo 100-8185 (JP); JAPAN as represented by DIRECTOR GENERAL OF AGENCY OF NACIONAL CANCER CENTER, Chuo-ku, Tokyo 104-0045 (JP)
(72) Inventor: OCHIAI, Atsushi,c/o Resear. Center for Inno.Onco., Kashiwa-shi, Chiba 2778577 (JP); KUSAKA, Hideaki, Kyowa HakkoKogyo Co., Ltd., Sunto-gun, Shizuoka 4118731 (JP); AKIYAMA, Tadakazu, Kyowa HakkoKogyo Co., Ltd., Shizuoka 4118731 (JP)
(74) Representative: Vossius & Partner
(86) International application number: PCT/JP2004/014452
(87) International publication number: WO 2005/027970

(57) **Abstract**

The invention aims to provide a medicament for treating cancer in which a cancer therapeutic effect is synergistically increased using a substance inhibiting activities of insulin-like growth factor-I (IGF-I) and insulin-like growth factor-II (IGF-II).

According to the invention, there are provided a medicament for treating cancer which comprises a substance inhibiting activities of IGF-I and IGF-II and which is administered in combination with irradiation; and a medicament for treating cancer comprising a combination of a substance inhibiting activities of IGF-I and IGF-II and a substance having an antitumor activity.

SEQUENCE LISTING

<110> KYOWA HAKKO KOGYO CO., LTD.

<110> NATIONAL CANCER CENTER

<120> MADICAMENT FOR TREATING CANCER

<130> 1000P11609WO0

<150> JP 2003-332346

<151> 2003-09-24

<160> 10

<170> PatentIn Ver. 2.0

<210> 1

<211> 18

<212> PRT

<213> Homo sapiens

<400> 1

Gly Pro Glu Thr Leu Ser Gly Ala Glu Leu Val Asp Ala Leu Gln Phe

1               5                   10                  15

Val Cys

<210> 2

<211> 18

<212> PRT

<213> Homo sapiens

<400> 2
Cys Leu Gln Phe Val Ala Gly Asp Arg Gly Phe Tyr Phe Asn Lys Pro
1               5                   10                  15

Thr Gly

<210> 3
<211> 13
<212> PRT
<213> Homo sapiens

<400> 3
Cys Tyr Phe Asn Lys Pro Thr Gly Tyr Gly Ser Ser Ser
1               5                   10

<210> 4
<211> 14
<212> PRT
<213> Homo sapiens

<400> 4
Cys Thr Gly Tyr Gly Ser Ser Ser Arg Arg Ala Pro Gln Thr
1               5                   10

<210> 5
<211> 12
<212> PRT
<213> Homo sapiens

<400> 5

Arg Arg Ala Pro Gln Thr Gly Ile Val Asp Glu Cys
1               5                   10

<210> 6

<211> 17

<212> PRT

<213> Homo sapiens

<400> 6

Cys Thr Gly Ile Val Asp Glu Ala Ala Phe Arg Ser Ala Asp Leu Arg
1               5                   10                  15

Arg

<210> 7

<211> 19

<212> PRT

<213> Homo sapiens

<400> 7

Cys Asp Leu Arg Arg Leu Glu Met Tyr Ala Ala Pro Leu Lys Pro Ala
1               5                   10                  15

Lys Ser Ala

<210> 8

<211> 9

<212> PRT

<213> Homo sapiens

<400> 8

Asp Leu Arg Arg Leu Glu Met Tyr Cys
1               5

<210> 9
<211> 10
<212> PRT
<213> Homo sapiens

<400> 9

Cys Ala Pro Leu Lys Pro Ala Lys Ser Ala
1               5                   10

<210> 10
<211> 17
<212> PRT
<213> Homo sapiens

<400> 10

Cys Thr Gly Ile Val Asp Glu Cys Cys Phe Arg Ser Cys Asp Leu Arg
1               5                   10                  15

Arg

## Description

### Technical Field

The present invention relates to a medicament for treating cancer which comprises a substance inhibiting activities of insulin-like growth factor-I (IGF-I) and insulin-like growth factor-II (IGF-II) and which is administered in combination with irradiation, and a medicament for treating cancer which comprises a combination of a substance inhibiting activities of insulin-like growth factor-I (IGF-I) and insulin-like growth factor-II (IGF-II) and a substance having an antitumor activity.

### Background Art

An insulin-like growth factor (hereinafter referred to as IGF) is a peptide hormone comprising a sequence of approximately 70 amino acids, having a structure similar to that of proinsulin and having three disulfide bonds in a molecule. IGF includes two types of peptides which are similar in structure and are called insulin-like growth factor-I (IGF-I) and insulin-like growth factor-II (IGF-II). IGF-I is synthesized and secreted mainly in the liver by stimulation of growth hormones, and plays an important role in growth stimulation of animals, such as promotion of chondrogenesis, protein synthesis, cell proliferation or cell differentiation. Meanwhile, IGF-II is involved in formation or development of organs in the fetal stage. A physiological activity of IGF is exhibited through an IGF receptor (hereinafter referred to as IGF-R) present in bones or muscles. Further, it is known that a protein called an IGF-binding protein (hereinafter referred to as IGFBP) exists to control functions of IGF in a promotive or inhibitive manner.

IGF-I and IGF-II both exhibit a strong growth promoting activity to a large number of cancer cells (sarcoma, leukemia, prostate cancer, breast cancer, lung cancer, stomach cancer, esophagal cancer, liver cancer, pancreas cancer, renal cancer, thyroid gland cancer, brain tumor, ovarian cancer and uterine cancer), and the over expression is observed in many cancer cells.

A relationship between IGF and cancers has been so far investigated in clinical and immunological studies, and it has been known that there is indeed a correlation between a cancer morbidity and an IGF concentration in blood. Accordingly, the IGF-family proteins (IGF, IGF-R and IGFBP) including IGF-I and IGF-II play an important role in onset and proliferation of cancers. Insulin and growth factors such as IGF-I and IGF-II are intricately entangled with an insulin receptor, IGF-IR, a receptor of IGF-IR and IGFBP to control diseases. That is, when only a part of these interactions are inhibited, it is difficult to suppress the diseases completely.

With regard to antibody to IGF or, in other words, anti-IGF antibody, some antibodies have been known already. As to a typical antibody to human IGF-I (anti-hIGF-I antibody), sml.2 has been reported *(Procedings of the National Academy of Sciences of the United States of America,* 81, 2389-2392, 1984). It has been became clear that sml.2 has a crossreactivity to hIGF-II of about 40%, can detect 100 ng of hIGF-I by a western blotting method at a concentration of 1 to 2 µg/ml and inhibits the proliferation of mouse fibroblast cell line BALB/c3T3 by 20 ng/mL of hIGF-I at a concentration of 10 to 30 µg/ml *(Procedings of he National Academy of Sciences of the United States of America,* 81, 2389-2392, 1984; *Journal of clinical Investigation,* 99, 2961-2970, 1997).

Besides the above, Val⁵⁹-SmC121 has been known as an anti-hIGF-I antibody and it has been reported that the antibody does not react with human insulin and hIGF-II, recognizes a peptide comprising Leu-Val-Asp exsiting at 10th to 12th position of hIGF and shows 1 ng/mL of hIGF-I detection sensitivity in a radioimmunoassay using ¹²⁵I-HIGF-I (*Journal of Endocrinology,* 125, 327-335, 1990). 41/81 has a reactivity of 3% to hIGF-II and, in a radioimmunoassay using ¹²⁵I-hIGF-I, it shows 1 ng/mL of hIGF-I detection sensitivity (*FEEBS Letters,* 149, 109-112, 1982). 35I17 has been reported to have a crossreactivity to hIGF-II of about 0.5%, to be able to detect 1 µg of hIGF-I by a western blotting method at a concentration of 1 µg/mL, to completely inhibit the proliferation of mouse fibroblast cell line BALB/c3T3 by hIGF-I in a concentration of not lower than 12 µg/mL, to inhibit a self-phosphorylation of hIGF-IR by 1 µg/mL of hIGF-I at a concentration of 30 µg/mL and to show 0.1 nM of hIGF-I detection sensitivity in a radioimmunoassay using ¹²⁵I-hIGF-I (*Hybridoma,* 16, 513-518, 1997). It has been reported that BPL-M23 shows a binding activity of 10.5 liters/nmol to hIGF-I while, to hIGF-II and human insulin, it shows crossreactivity of 0.8% and 0.0001%, respectively, that, although it shows reactivity to IGF of goat, pig, sheep, cattle and rabbit, it does not react with IGF of rat and mouse and that it suppresses a fat formation by hIGF-1 in fat cells of rat *(Journal of Molecular Endocrinology,* 2, 201-206, 1989). 7A1, 1B3, 4C1 and 5A7 have been reported to recognize different epitopes of C and D domains of hIGF-1 and to show crossreactivity to hIGF-II of 6.6%, 0.83%, 12% and 1.2%, respectively (*Hybridoma,* 12, 737-744, 1993). 3D1/2/1 has been reported that, although it shows reactivity with IGF-I of human and guinea pig, it does not react with IGF-I of rabbit, rat and mouse and that it shows crossreactivity of 7% with hIGF-II *(Journal of Clinical and Metabolism,* 54, 474-476, 1982).

With regard to a typical antibody to human IGF-II (anti-hIGF-II antibody), S1F2 has been reported. It has been revealed that S1F2 has crossreactivity of about 10% with hIGF-I, that it can detect 10 to 100 ng of hIGF-II by a western blotting method at a concentration of 1 µg/mL and that it inhibits the promoting activity for the DNA synthesis of human fibroblast cells by 100 ng/mL of hIGF-II at a concentration of 100 µg/mL *(Diabetes Research and Clinical Practice,* 7, S21-S27, 1989 ; *Endocrinology,* 124, 870-877, 1989). It has been reported that 2H11, 2B11, ID5 and ID9 react with hIGF-II, do not react with hIGF-I and can determine 1 ng/mL of hIGF-II by a competitive enzyme immunoassay (hereinafter, referred to as ELISA) (Japanese Published Unexamined Patent Application No. 252987/93). However, an antibody which binds to both of insulin-like growth factor-I (IGF-I) and insulin-like growth factor-II (IGF-II) and which inhibits both activities has not been known.

Further, the effect of combination therapy of an antibody having the above-mentioned property with other cancer therapy has not been known.

### Disclosure of the Invention

The object of the present invention is to provide a medicament for treating cancer and a method for treating cancer in which a therapeutic effect for cancer is synergistically increased using a substance inhibiting activities of insulin-like growth factor-I (IGF-I) and insulin-like growth factor-II (IGF-II).

The present inventors have assiduously conducted investigations to solve the above problems, and have consequently found that a cancer therapeutic effect can be increased using a substance inhibiting activities of insulin-like growth factor-I (IGF-I) and insulin-like growth factor-II (IGF-II) in combination with irradiation or a substance having an antitumor activity, and the present invention was completed.

Thus, the present invention includes the following inventions (1) to (15).
(1) A medicament for treating cancer which comprises a substance inhibiting activities of insulin-like growth factor-I (IGF-I) and insulin-like growth factor-II (IGF-II) and which is administered in combination with irradiation.
(2) The medicament for treating cancer according to (1), wherein the irradiation is conducted once or plural times at the time of administrating the medicament for treating cancer, or before or after the administration.
(3) A medicament for treating cancer which comprises a combination of a substance inhibiting activities of insulin-like growth factor-I (IGF-I) and insulin-like growth factor-II (IGF-II) and a substance having an antitumor activity.
(4) The medicament for treating cancer according to (3), wherein the substance inhibiting the activities of insulin-like growth factor-I (IGF-I) and insulin-like growth factor-II (IGF-II) and the substance having the antitumor activity are administered simultaneously or consecutively.
(5) The medicament for treating cancer according to any one of (1) to (4), wherein the substance inhibiting the activities of insulin-like growth factor-I (IGF-I) and insulin-like growth factor-II (IGF-II) is selected from the group consisting of the following (a) to (d),
   (a) an antibody or an antibody fragment which is specifically binds to IGF-I and IGF-II to inhibit the activities of IGF-I and IGF-II,
   (b) a composition comprising an antibody or an antibody fragment which is specifically binds to IGF-I to inhibit the activity of IGF-I and an antibody or an antibody fragment which specifically binds to IGF-II to inhibit the activity of IGF-II,
   (c) a component wherein an antibody or an antibody fragment which specifically binds to IGF-I to inhibit the activity of IGF-I and an antibody or an antibody fragment which specifically binds to IGF-II to inhibit the activity of IGF-II are combined, and
   (d) a complex of an antibody or an antibody fragment which specifically binds to IGF-I to inhibit the activity of IGF-I and an antibody or an antibody fragment which specifically binds to IGF-II to inhibit the activity of IGF-II.
(6) The medicament for treating cancer according to (5), wherein the antibody is a monoclonal antibody.
(7) The medicament for treating cancer according to (6), wherein the monoclonal antibody is a monoclonal antibody which binds to an epitope to which a monoclonal antibody produced from hybridoma KM 1468 (FERM BP-7978) binds.
(8) The medicament for treating cancer according to any one of (5) to (7), wherein the antibody fragment is an antibody fragment selected from the group consisting of Fab, Fab', F(ab')₂, a single chain antibody (scFv), a dimeric variable region (Diabody), a disulfide stabilized variable region (dsfv) and a CDR-containing peptide.
(9) The medicament according to (1) to (8), wherein the substance having the antitumor activity is a protein or an agent having low-molecular weight.
(10) The medicament according to (9), wherein the protein is an antibody or a cytokine.
(11) The medicament according to (9), wherein the agent having low-molecular weight is an agent selected from the group consisting of a DNA alkylating agent, a DNA synthesis inhibitor, a platinum preparation-type DNA crosslinking agent, a metabolic antagonist, a topoisomerase I inhibitor, a topoisomerase II inhibitor, a tubulin acting agent, a hormone antagonist, an aromatase inhibitor, an immunomodulator, an immunosuppressant, a steroidal antiinflammatory agent, a non-steroidal antiinflammatory agent, an antihistaminic agent, a differentiation inducer, a proteasome inhibitor, a tyrosine kinase inhibitor, an adenosine deaminase inhibitor, an angiogenesis inhibitor, a histone deacetylase inhibitor, a matrix metalloproteinase inhibitor, a farnesyl transferase inhibitor, a bisphosphonate preparation, an Hsp90 inhibitor, a kinesin Eg5 inhibitor, a serine threonine kinase inhibitor and derivatives of these compounds.
(12) A method for treating cancer which comprises administering to a mammal an effective amount of a substance inhibiting activities of insulin-like growth factor-I (IGF-I) and insulin-like growth factor-II (IGF-II) in combination with irradiation.
(13) The method for treating cancer according to (12), wherein the irradiation is conducted once or plural times at the time of administering a medicament for treating cancer, or before or after the administration.
(14) A method for treating cancer which comprises administering to a mammal an effective amount of a substance inhibiting activities of insulin-like growth factor-I (IGF-I) and insulin-like growth factor-II (IGF-II) and an effective amount of a substance having an antitumor activity in combination.
(15) The method for treating cancer according to (14), wherein the effective amount of the substance inhibiting the activities of insulin-like growth factor-I (IGF-I) and insulin-like growth factor-II (IGF-II) and the effective amount of the substance having the antitumor activity are administered simultaneously or successively.

### Brief Description of the Drawings

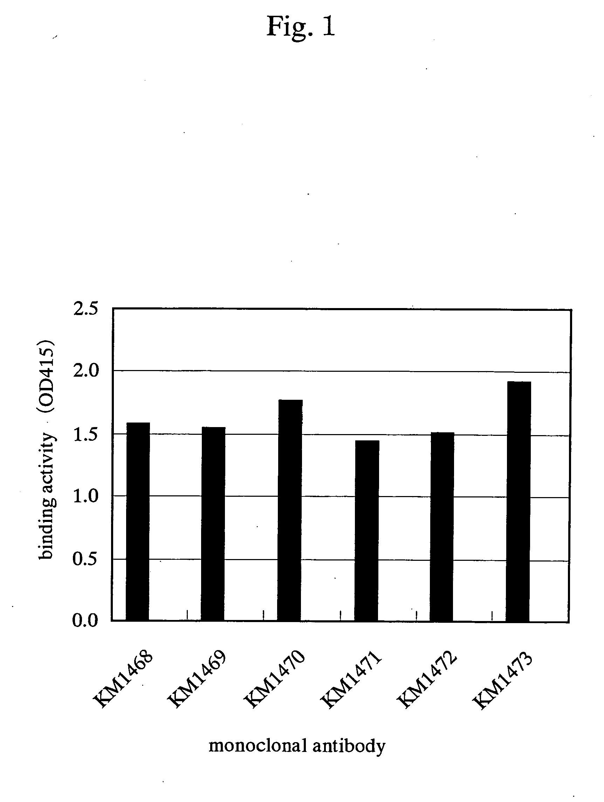

Fig. 1 shows the specific reactivity of a monoclonal antibody to hIGF-I (binding ELISA). The abscissa shows combination of antibody with antigen while the ordinate shows binding activity (OD415).

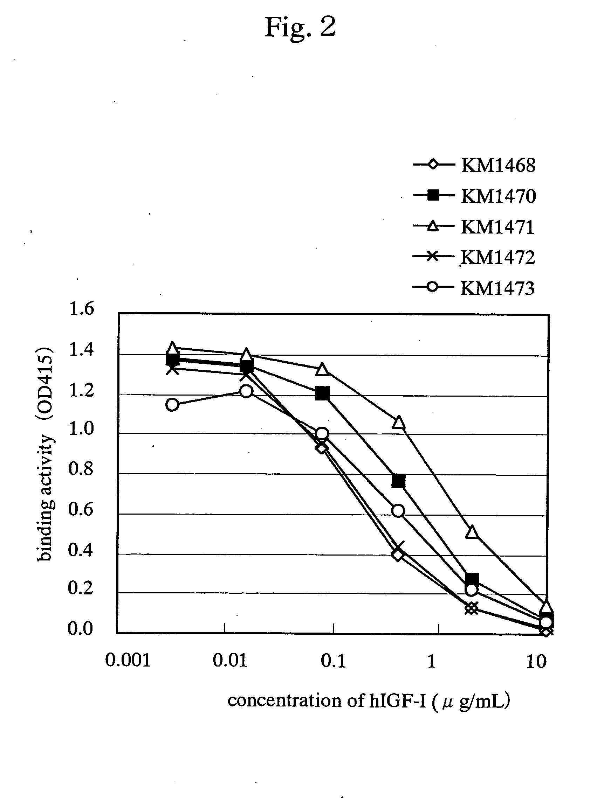

Fig. 2 shows reactivity of a monoclonal antibody to hIGF-I having an natural three-dimensional structure in a liquid phase system (competitive ELISA). The abscissa shows concentration of the hIGF-I added while the ordinate shows binding activity (OD415).

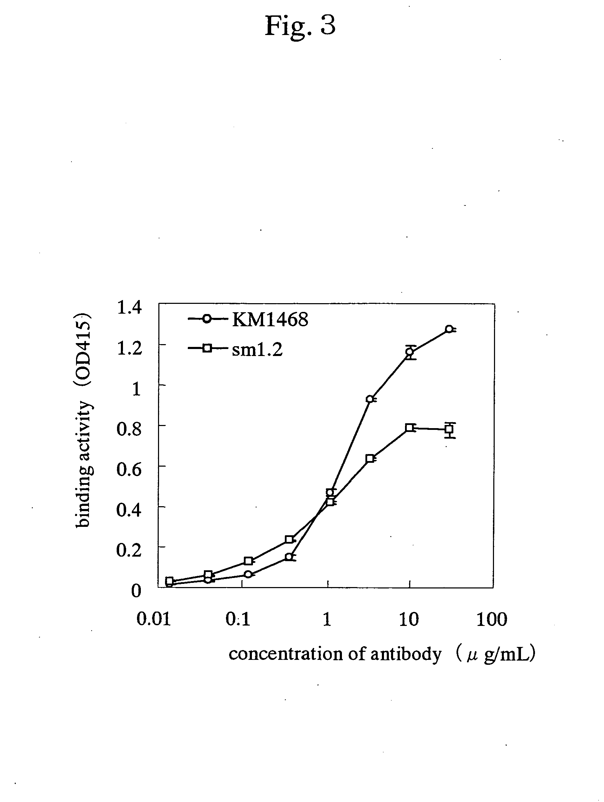

Fig. 3 shows the reactivity of the antibody KM 1468 and sm1.2 to hIGF-I. The abscissa shows concentration of the antibody (µg/mL) while the ordinate shows binding activity (OD415). Open-circular and open-square show reactivity of KM 1468 and that of sm1.2, respectively.

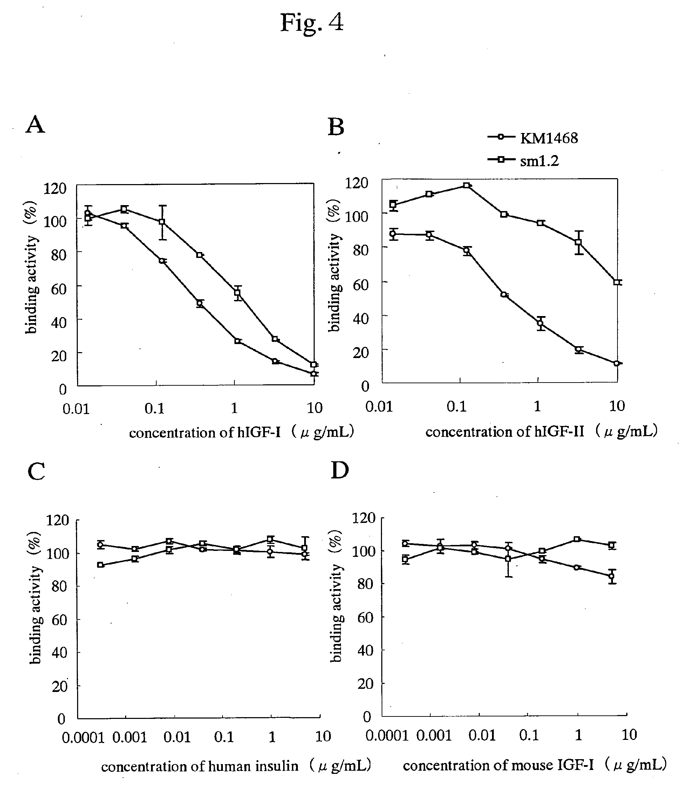

Fig. 4 shows inhibitory activities of various factors to the binding of the antibody KM1468 and sml.2 to hIGF-I . The abscissa shows concentration of each factor while the ordinate shows binding activity (%). A, B, C and D show activities by hIGF-I, hIGF-II, human insulin and mIGF-I, respectively. Open-circular and open-square show reactivity of KM 1468 and that of sm1.2, respectively.

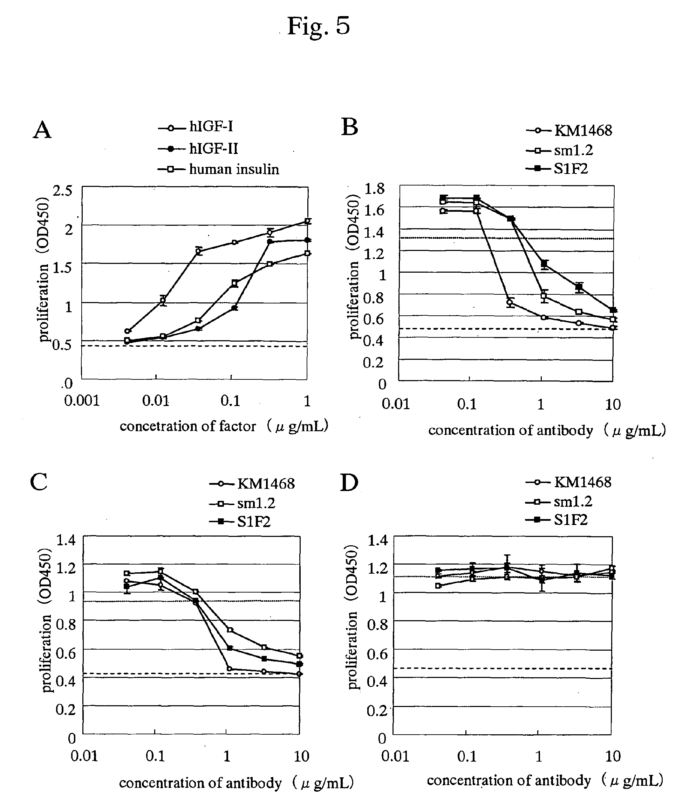

Fig. 5 shows influences of the antibodies KM 1468, sm1.2 and S1F2 on the proliferation of a human breast cancer cell line MCF7 by hIGF and human insulin. "A" shows a cell proliferation activity by each factor. The abscissa shows concentration of each factor (µg/mL) while the ordinate shows the proliferation (OD450). Open-circular, closed-circular and open-square show activities of hIGF-I, hIGF-II and human insulin, respectively. B, C and D show influences of various antibodies on proliferation activity by hIGF-I, hIGF-II and human insulin, respectively. The abscissa shows concentration of antibody (µg/mL) while the ordinate shows the proliferation (OD450). A dotted line shows the proliferation in the absence of antibody while a broken line shows the proliferation in the absence of each factor. Open-circular, open-square and closed-square show activities of KM 1468, sm1.2 and S1F2, respectively.

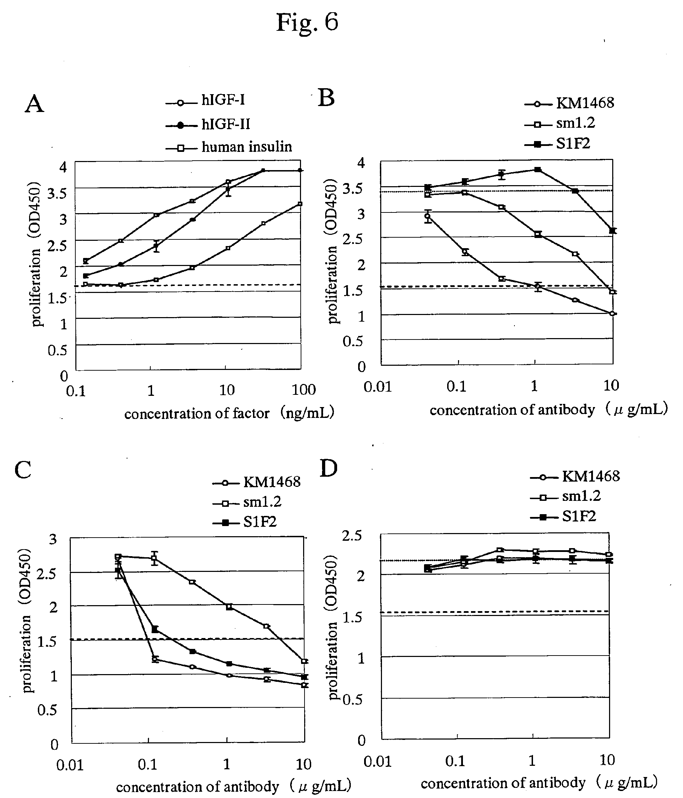

Fig. 6 shows influences of the antibodies KM 1468, sm1.2 and S1F2 on the proliferation of a human colorectal cancer cell line HT-29 by hIGF and human insulin. "A" shows a cell proliferation activity by each factor. The abscissa shows concentration of each factor (ng/mL) while the ordinate shows the proliferation (OD450). Open-circular, closed-circular and open -square show activities of hIGF-I, hIGF-II and human insulin, respectively. B, C and D show influences of various antibodies on proliferation activity by hIGF-I, hIGF-II and human insulin, respectively. The abscissa shows concentration of antibody (µg/mL) while the ordinate shows the proliferation (OD450). A dotted line shows the proliferation in the absence of antibody while a broken line shows the proliferation in the absence of each factor. Open-circular, open-square and closed-square show activities of KM 1468, sml.2 and S1F2, respectively.

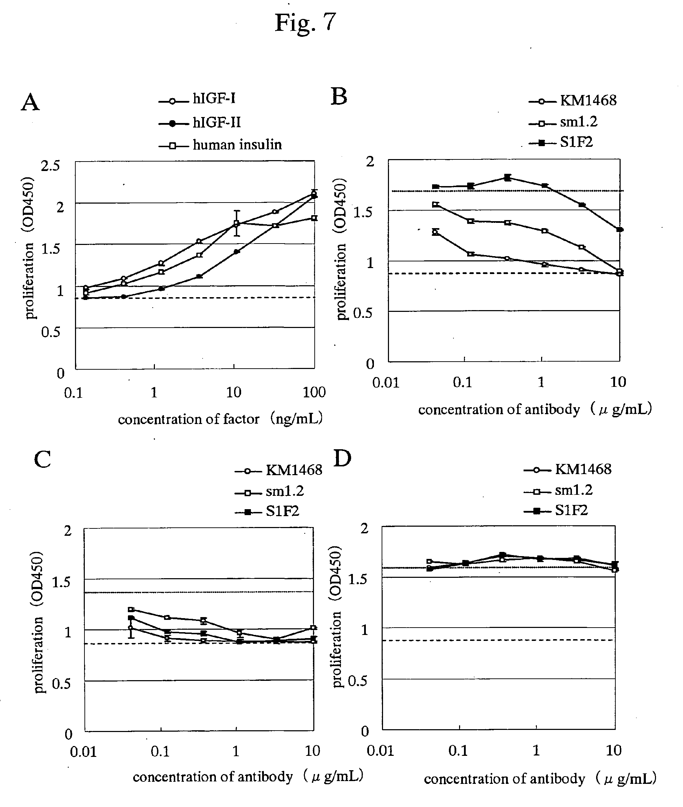

Fig. 7 shows influences of the antibodies KM 1468, sml.2 and S1F2 on the proliferation of a human osteosarcoma cell line MG63 by hIGF and human insulin. "A" shows a cell proliferation activity by each factor. The abscissa shows concentration of each factor (ng/mL) while the ordinate shows the proliferation (OD450). Open-circular, closed-circular and open -square show activities of hIGF-I, hIGF-II and human insulin, respectively. B, C and D show influences of various antibodies to proliferation activity by hIGF-I, hIGF-II and human insulin, respectively. The abscissa shows concentration of antibody (µg/mL) while the ordinate shows the proliferation (OD450). A dotted line shows proliferation in the absence of antibody while a broken line shows proliferation in the absence of each factor. Open-circular, open-square and closed-square show activities of KM 1468, sm1.2 and S1F2, respectively.

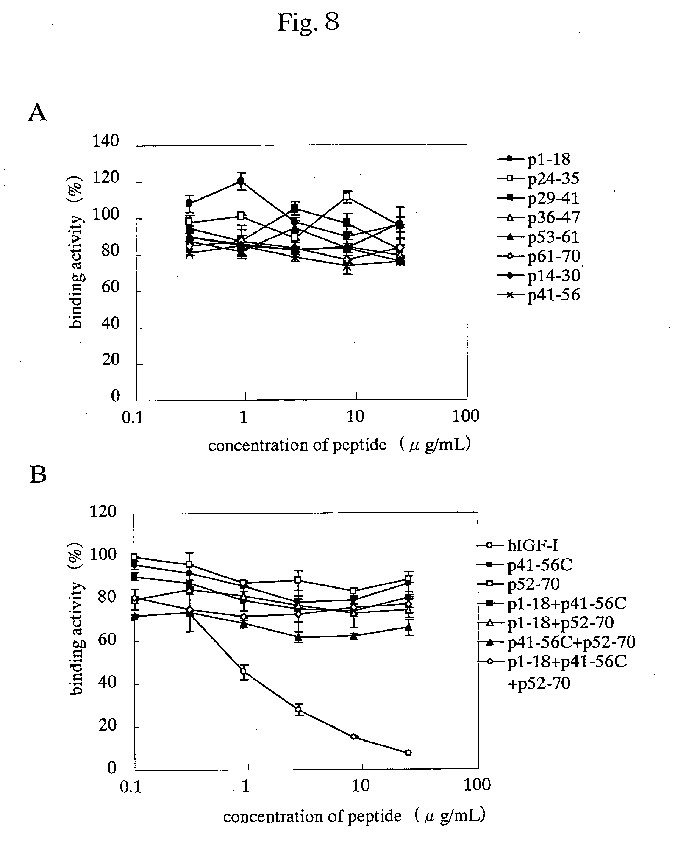

Fig. 8 shows inhibitory activities to various peptides in binding of an antibody KM 1468 to hIGF-I . The abscissa shows concentration of various peptide (µg/mL) while the ordinate shows binding activity (%). The various peptides used are mentioned in the drawings.

The present invention is described in detail below. The present application claims Convention priority from Japanese Patent Application No. 2003-332346 filed on September 24, 2003, and it includes the contents described in the specification and/or drawings of the present application.

### I. Medicament for treating cancer and method for treating cancer of the present invention

The medicament for treating cancer of the present invention is a medicament for treating cancer which comprises a substance inhibiting activities of insulin-like growth factor-I (IGF-I) and insulin-like growth factor-II (IGF-II) and which is administered in combination with irradiation.

The medicament for treating cancer of the present invention is further a medicament for treating cancer which comprises a combination of a substance inhibiting activities of insulin-like growth factor-I (IGF-I) and insulin-like growth factor-II (IGF-II) and a substance having an antitumor activity.

In the medicament for treating cancer of the present invention, "the substance which inhibits the activities of IGF-I and IGF-II" may be a single substance or may be a composition comprising plural substances and, in the case of a composition comprising plural substances, each of the substances may be used either simultaneously or separately.

Examples of the substance which inhibits the activities of IGF-I and IGF-II are
(a) an antibody or an antibody fragment which specifically binds to IGF-I and IGF-II and inhibits the activity of IGF-I and IGF-II;
(b) a composition comprising an antibody or an antibody fragment which specifically binds to IGF-I and inhibits the activity of IGF-I and an antibody or an antibody fragment which specifically binds to IGF-II and inhibits the activity of IGF-II;
(c) a component wherein an antibody or an antibody fragment which specifically binds to IGF-I and inhibits the activity of IGF-I and an antibody or an antibody fragment which specifically binds to IGF-II and inhibits the activity of IGF-II are combined; and
(d) a complex of an antibody or an antibody fragment which specifically binds to IGF-I and inhibits the activity of IGF-I and an antibody or an antibody fragment which specifically binds to IGF-II and inhibits the activity of IGF-II.

The aforementioned expression of "to inhibit the activity of IGF-I and IGF-II" means that any of the activities of IGF-I and IGF-II is inhibited and its specific example is to inhibit the activity of promoting the cell proliferation by IGF-I and IGF-II.

The expression of "an antibody or an antibody fragment which specifically binds to IGF-I and IGF-II and inhibits the activity of IGF-I and IGF-II" used in the present invention means an antibody or an antibody fragment which specifically binds to both IGF-I and IGF-II and inhibits the activities of both IGF-I and IGF-II. Specific examples thereof are an antibody or an antibody fragment which recognizes the epitope existing in an natural IGF-I and an natural IGF-II, an antibody or an antibody fragment which recognizes the three-dimensional structure of IGF-I and IGF-II, and the like.

Although aforementioned antibody or the antibody fragment used in the present invention may be any of a polyclonal antibody or a monoclonal antibody, a monoclonal antibody is preferred. Further, a monoclonal antibody includes "a hybridoma-producing antibody", "a recombinant antibody" and antibody fragments thereof, and the like. Examples of the "recombinant antibody" are a humanized antibody, a human antibody, and the like and examples of the "humanized antibody" are a human chimeric antibody, a human complementarity determining region (hereinafter referred to as CDR)-grafted antibody. A "hybridoma" is a cell which is obtained by cell fusion between a B cell obtained by immunizing a non-human mammal with an antigen and a myeloma cell and can produce a monoclonal antibody having the desired antigen specificity.

The "human chimeric antibody" means an antibody comprising heavy chain variable region (hereinafter referred to as VH) and light chain variable region (hereinafter referred to as VL) of an antibody of the non-human animal and heavy chain constant region (hereinafter referred to as CH) and light chain constant region (hereinafter referred to as CL) of a human antibody. As CH of the human chimeric antibody, although any CH may be used so long as it belongs to human immunoglobulin (hereinafter, referred to as hIg), that of an hIgG class is preferred and any of subclasses hIgG1, hIgG2, hIgG3 and hIgG4 belonging to an hIgG class may be used as well. With regard to CL of a human chimeric antibody, any CL may be used so long as it belongs to hIg and any of a κ class and a λ class may be used. The non-human animals are mouse, rat, hamster, rabbit and the like.

The "human CDR-grafted antibody" means an antibody in which CDRs of VH and VL of an antibody of the non-human animal are grafted to an appropriate positions of VH and VL of a human antibody. The human CDR-grafted antibody according to the present invention can be produced by designing and constructing cDNAs coding for a V region in which CDRs of VH and VL of the non-human animal is ligated with a framework (hereinafter, referred to as FR) of VH and VL of any human antibody, inserting them respectively into expression vector for animal cell having cDNAs coding for CH and CL of a human antibody to construct a human CDR-grafted antibody expression vector followed by introducing the expression vector into animal cell for expression.

With regard to CH for a human CDR-grafted antibody, although any CH may be used so long as it belong to hIg, that of an hIgG class is preferred and any of subclasses hIgG1, hIgG2, hIgG3 and hIgG4 belonging to an hIgG class may be used as well. With regard to CL of a human CDR-grafted antibody, any CL may be used so long as it belongs to hIg and that of a κ class and a λ class may be used.

Although the "human antibody" generally means an antibody naturally existing in human body, it also includes an antibody which is prepared from a human antibody phage library and from human antibody-producing transgenic animals prepared by the recent progresses in techniques in genetic engineering, cell engineering and developmental engineering. Regarding the antibody existed in the human body, for example, a lymphocyte capable of producing said antibody can be cultured by isolating a human peripheral lymphocyte, immortalizing by infecting with EB virus or the like and then cloning, and said antibody can be purified from the culture supernatant. The human antibody phage library is a library in which antibody fragments of Fab, scFv and the like are expressed on the phage surface by inserting an antibody gene prepared from human B cell into a phage gene. A phage expressing antibody fragments having the desired antigen binding activity on the surface can be recovered from said library using the binding activity to an antigen-immobilized substrate as an index. Said antibody fragments can be further converted into a human antibody molecule comprising two full length H chains and two full length L chains by genetic engineering techniques. The human antibody-producing transgenic animal means an animal in which a human antibody gene is integrated into its cells. For example, a human antibody-producing transgenic mouse can be prepared by introducing a human antibody gene into a mouse ES cell, transplanting said ES cell into early embryo of a mouse and then developing. Regarding the method for preparing a human antibody from a human antibody-producing transgenic animal, the human antibody can be produced and accumulated in a culture supernatant by culturing a human antibody-producing hybridoma obtained by a hybridoma preparation method generally carried out in non-human animal.

The antibody or the antibody fragment which is preferably used in the present invention includes a monoclonal antibody KM 1468 produced by hybridoma KM 1468 (FERM BP-7978), a monoclonal antibody binding to an epitope to which a monoclonal antibody KM 1468 produced by hybridoma KM 1468 (FERM BP-7978) binds or an anti-hIGF-I monoclonal antibody sm1.2 (Upstate Biology) which reacts with hIGF-1 and also have about 40% crossreactivity with hIGF-II, an monoclonal antibody binding to an epitope to which anti-hIGF-I monoclonal antibody sm1.2 (Upstate Biology) binds, and further, recombinant antibodies or antibody fragments comprising amino acid sequences of CDR1, CDR2 and CDR3 of VH and VL of the foregoing monoclonal antibodies, and the like, are exemplified. With regard to "a composition comprising the antibody or the antibody fragment which specifically binds to IGF-I and inhibits the activity of IGF-I and the antibody or the antibody fragment which specifically binds to IGF-II and inhibits the activity of IGF-II", any composition may be used so long as it is a composition comprising the antibody or the antibody fragment which inhibits the activity of IGF-I and the antibody or the antibody fragment which inhibits the activity of IGF-II.

The expression of "the antibody or the antibody fragment which specifically binds to IGF-I and inhibits the activity of IGF-I" means an antibody which specifically binds to IGF-I but does not specifically bind to IGF-II (having no crossreactivity) and its examples are AF791 (manufactured by R & D) which is an antibody to mouse IGF-I (hereinafter, referred to as mIGF-1), 56408 (manufactured by R & D) which is a monoclonal antibody to human IGF-I (hereinafter, mentioned as hIGF-I), M23/ILG1-001 (manufactured by Biogenesis), an monoclonal antibody binding to an epitope to which AF791 binds, an monoclonal antibody binding to an epitope to which 56408 binds, an monoclonal antibody binding to an epitope to which M23/ILG1-001 binds, and further includes recombinant antibodies or antibody fragments comprising amino acid sequences of CDR1, CDR2 and CDR3 of VH and VL of the foregoing monoclonal antibodies, and the like.

The expression of "the antibody or the antibody fragment which specifically binds to IGF-II and inhibits the activity of IGF-II" means an antibody which specifically binds to IGF-II but does not specifically bind to IGF-I (having no crossreactivity) and its examples are AF792 (manufactured by R & D) which is an antibody to mIGF-II, S1F2 (manufactured by Upstate Biology) which is a monoclonal antibody to hIGF-II, an monoclonal antibody binding to an epitope to which AF792 binds, an monoclonal antibody binding to an epitope to which S1F2 binds, an monoclonal antibody binding to an epitope to which M23/ILG1-001 binds, and further includes gene recombinant antibodies or antibody fragments comprising amino acid sequences of CDR1, CDR2 and CDR3 of VH and VL of the foregoing monoclonal antibodies, and the like.

The expression of "a component wherein an antibody or an antibody fragment which specifically binds to IGF-I and inhibits the activity of IGF-I and an antibody or an antibody fragment which specifically binds to IGF-II and inhibits the activity of IGF-II are combined" is a composition in which an medicament comprising "the antibody or the antibody fragment which specifically binds to IGF-I and inhibits the activity of IGF-I" and an medicament comprising "the antibody or the antibody fragment which specifically binds to IGF-II and inhibits the activity of IGF-II" are separately prepared and those medicaments are combined for a simultaneous use or a successive use.

With regard to "a complex of an antibody or an antibody fragment which specifically binds to IGF-I and inhibits the activity of IGF-I and an antibody or an antibody fragment which specifically binds to IGF-II and inhibits the activity of IGF-II", any complex may be used so long as it is a complex prepared by binding the antibody or the antibody fragment inhibiting the activity of IGF-I and the antibody or the antibody fragment inhibiting the activity of IGF-II. To be more specific, an antibody complex in which the two kinds of antibodies or antibody fragments are bound by the following methods may be exemplified.

With regard to a method for binding the antibodies, a method in which they are chemically bound and a method using protein engineering may be exemplified.

With regard to a method where they are chemically bound, a method in which two kinds of antibody molecules are bound using a cross-linking agent such as N-succinimidyl-3-(2-pyridyldithiol) propionate and S-acetylmercaptosuccinic acid anhydride may be exemplified.

With regard to a method of binding using a protein engineering techniques, any method may be used so long as it is a method in which plural antibodies or antibody fragments can be expressed in a complex using a protein engineering techniques. Examples of the antibody complex prepared by a method of binding using a protein engineering techniques are a molecule in which two kinds of scFv are linked via an appropriate linker, a molecule where two kinds of antibody Fab' fragments are bound via an appropriate linker, an Fc fused protein where two kinds of scFv are bound to N-terminal and C-terminal, a heteromolecule of Fc fused protein where two kinds of scFv are bound, diabody and an Fc fused protein in which diabody is bound to N-terminal or C-terminal.

Examples of the antibody fragment used in the present invention include Fab, Fab', F(ab')₂, scFv, diabody, dsFv, CDR-containing peptide, and the like.

Fab is an antibody fragment having a molecular weight of about 50,000 and having an antigen-binding activity where about one half of N-terminal side of H chain and the full length of L chain, among fragments obtained by treating IgG-type antibody molecule with a protease, papain (cleaving an amino acid residue at position 224 of an H chain) are bound together through a disulfide bond.

The Fab used in the present invention can be prepared by treating the antibody with a protease, papain. Alternatively, DNA which codes for Fab of the antibody is inserted into expression vector for prokaryote or expression vector for eukaryote and the vector is introduced into prokaryote or eukaryote to express whereupon Fab is produced.

F(ab')₂ is an antibody fragment having a molecular weight of about 100,000 and having an antigen-binding activity which is slightly larger than the Fab bound via disulfide bond of the hinge region, among fragments obtained by treating IgG-type antibody molecule with a protease, pepsin.

The F(ab')₂ used in the present invention can be prepared by treating the antibody with a protease, pepsin. Alternatively, it can be prepared by linking Fab' described below via a thioether bond or a disulfide bond.

Fab' is an antibody fragment having a molecular weight of about 50,000 and exhibiting an antigen binding activity where a disulfide bond at a hinge region of the aforementioned F(ab')₂ is cleaved.

The Fab' used in the present invention can be prepared by treating F(ab')₂ with a reducing agent, dithiothreitol. Alternatively, DNA which codes for Fab' fragment of the antibody is inserted into expression vector for prokaryote or expression vector for eukaryote and the vector is introduced into the prokaryote or the eukaryote to express whereupon Fab' can be prepared.

scFv is an antibody fragment having an antigen binding activity and is an VH-P-VL or an VL-P-VH polypeptide where one VH and one VL are linked using an appropriate peptide linker (hereinafter, referred to as P).

The scFv used in the present invention can be prepared in such a manner that cDNA coding for VH and VL of the antibody is obtained, DNA coding for scFV is constructed, the DNA is inserted into expression vector for prokaryote or expression vector for eukaryote and the expression vector is introduced into the prokaryote or the eukaryote to express whereupon the scFc can be prepared.

Diabody is an antibody fragment where svFv is dimerized and is an antibody fragment having divalent antigen binding activity. The divalent antigen binding activity may be the same or one of them can be used as a different antigen binding activity. The diabody used in the present invention can be prepared in such a manner that cDNA coding for VH and VL of antibody is obtained, DNA coding for scFv is constructed so as to make the length of amino acid sequence of the linker to be not more than 8 residues, the DNA is inserted into expression vector for prokaryote or expression vector for eukaryote and the expression vector is introduced into the prokaryote or the eukaryote to express whereupon the diabody can be prepared.

The dsFv is an antibody fragment where a polypeptide in which each one amino acid residue in VH and VL is substituted with a cysteine residue is linked via a disulfide bond between the cysteine residues. The amino acid residue to be substituted with a cysteine residue can be selected based on a three-dimensional structure estimation of the antibody according to a method shown by Reiter, et al. *(Protein Engineering,* 7, 697-704, 1994). The dsFv used in the present invention can be prepared in such a manner that cDNA coding for VH and VL of an antibody is obtained, DNA coding for the dsFv is constructed, the DNA is inserted into expression vector for prokaryote or expression vector for eukaryote and the expression vector is introduced into the prokaryote or the eukaryote to express whereupon the dsFv can be prepared.

A CDR-containing peptide is constituted by comprising at least one region of CDRs of VH or VL. A peptide comprising plural CDRs can be linked either directly or via an appropriate peptide linker. A CDR-containing peptide used in the present invention can be prepared in such a manner that DNA coding for VH and VL of an antibody is obtained, the DNA is inserted into expression vector for prokaryote or expression vector for eukaryote and the expression vector is introduced into the prokaryote or the eukaryote to express whereupon the CDR-containing peptide is prepared. The CDR-containing peptide can also be prepared by a chemical synthetic method such as an Fmoc method (fluorenylmethyloxycarbonyl method) and a tBoc method (tert-butyloxycarbonyl method).

With regard to the antibody or the antibody fragment of the present invention, it is possible to evaluate a binding activity to IGF-I and IGF-II and an activity inhibiting the activity of IGF-I and IGF-II *in* vitro by ELISA (Antibodies: A Laboratory Manual, Cold Spring Harbor Laboratory, Chapter 14, 1988; Monoclonal Antibodies: Principles and Practice, Academic Press Limited, 1996) and by measuring an inhibitory activity of cell proliferation by IGF-I and IGF-II (*Cancer Research*, 48, 4083-4092, 1988), and the like. In one embodiment of the present invention, when the irradiation is used in combination with the substance inhibiting activities of IGF-I and IGF-II, the above irradiation can be conducted either once or plural times at the time of administering the medicament comprising the substance, or before or after the administration. Further, the exposure dose of one irradiation is from 1 Gy to 10 Gy, preferably from 2 Gy to 5 Gy, more preferably 4 Gy. When the irradiation is conducted plural times, the exposure dose of one irradiation may be divided into smaller exposure doses.

In the present invention, the irradiation refers to a wide concept including exposure of photons (electromagnetic waves) such as X rays and γ rays, and exposure of particle rays such as electron rays, proton rays and heavy particle rays.

In another embodiment of the present invention, when the substance having the antitumor activity is used in combination with the substance inhibiting activities of IGF-I and IGF-II, the substance having the antitumor activity includes proteins, agents having low-molecular weight and the like.

The "antitumor activity" includes an activity of selective growth inhibition or damage of tissues or cells of malignant tumors, and an activity of reduction or disappearance of tumor tissues or cells. It is thus interpreted in the broadest sense.

The proteins are not limited. Examples thereof include antibodies, cytokines or the like.

Examples of the cytokines include interferons-α, -β and -γ, tumornecrosis factor (TNF)-α, lymphotoxin, interleukins-1, -2, -3, -4, -7, -8, -12, -15, -18 and -21, granulocyte-colony stimulating factor (G-CSF), macrophage colony stimulating factor (M-CSF), granulocyte-macrophage colony stimulating factor (GM-CSF), interferon-γ inducing protein-10 (IP-10), fractalkine and the like. Further, protein pharmaceutical preparations such as a growth hormone receptor antagonist, and the like are also included therein.

Regarding the antibodies, any antibodies against antigens expressed in tumor cells or antigens involved in formation of tumor pathogenic states such as growth and metastasis of tumor cells may be used. Examples thereof include antibodies against interleukin-6 (IL-6) receptor, GD2, GD3, GM2, HER2, CD20, CD22, CD33, CD52, MAGE, HM1.24, parathyroid hormone-related protein (PTHrP), basic fibroblast growth factor, fibroblast growth factor 8, basic fibroblast growth factor receptor, fibroblast growth factor 8 receptor, epithelial cell growth factor receptor (EGFR), epithelial cell adhesion molecule (EpCam), insulin-like growth factor, insulin-like growth factor receptor, PMSA, vascular endothelial cell growth factor (VEGF), vascular endothelial cell growth factor receptor (VEGFR) and the like.

Specific examples of the antibodies do not limit the scope of the invention. The anti-IL-6 receptor antibody includes those described in Anticancer Res., 18, 1217 (1998), the anti-GD2 antibody includes those described in Anticancer Res. 13, 331 (1993), the anti-GD3 antibody includes those described in Cancer Immunol. Immunother., 36, 260 (1993), the anti-GM2 antibody includes those described in Cancer Res., 54, 1511 (1994), the anti-HER2 antibody includes those described in Proc. Natl. Acad. Sci. USA, 89, 4285 (1992), the anti-CD20 antibody includes those described in Blood, 83, 435 (1994), the anti-CD22 antibody includes those described in Semmin. Oncol., 30, 253 (2003), the anti-CD33 antibody includes those described in J. Clin. Oncol., 19, 3244 (2001), the anti-CD52 antibody includes those described in Proc. Natl. Acad. Sci. USA, 89, 4285 (1992), the anti-MAGE antibody includes those described in British J. Cancer, 83, 493 (2000), the anti-HM1.24 antibody includes those described in Molecular Immunol., 36, 387 (1999), the anti-parathyroid hormone-related protein (PTHrP) antibody includes those described in Cancer, 88, 2909 (2000), the anti-fibroblast growth factor 8 antibody includes those described in Proc. Natl. Acad. Sci. USA, 86, 9911 (1989), the anti-fibroblast growth factor 8 receptor antibody includes those described in J. Biol. Chem., 265, 16455 (1990), the anti-epidermal cell growth factor receptor antibody includes those described in Cancer Res., 59, 1236 (1999), the anti-epidermal cell adhesion molecule antibody includes those described in Proc. Natl. Acad. Sci. USA, 76, 1438 (1979), the anti-insulin-like growth factor antibody includes those described in J. Neurosci. Res., 40, 647 (1995), the anti-insulin-like growth factor receptor antibody includes those described in J. Neurosci. Res. 40, 647 (1995), the anti-PMSA antibody includes those described in J. Urology, 160, 2396 (1998), the anti-vascular endothelial cell growth factor antibody includes those described in Cancer Res. 57, 4593 (1997), and the anti-vascular endothelial cell growth factor receptor antibody includes those described in Oncogene, 19, 2138 (2000).

Specific antibody names are Herceptin, Rituxan, Campath, Avastin, Bexxar, LymphoCide, Mylotarg, Panorex, Zevalin [Nat. Rev. Cancer, 1, 118 (2001)], and the like.

The agent having low-molecular weight is not limited. Examples thereof include DNA alkylating agents such as cyclophosphamide, ifosfamide, melphalan, dacarbazine, procarbazine, nimustin, carmustin, lomustine, estramustine, busulfan and thiotepa; DNA synthesis inhibitors such as bleomycin, peplomycin, mitomycin C and mitoxantrone; platinum preparation-type DNA crosslinking agents such as cisplatin, carboplatin, oxaliplatin and nedaplatin; anti-metabolites such as 5-fluorouracil, capecitabine, methotrexate, gemcitabine, fludarabine, cytarabine, cladribine, mercaptopurine, hydroxycarbamide and Ara-C; topoisomerase I inhibitors such as irinotecan and nogitecan; topoisomerase II inhibitors such as doxorubicin, epirubicin, daunorubicin and etoposide; tubulin acting agents such as vincristine, vinblastine, vindesine, vinorelbin, paclitaxel and docetaxel; hormone antagonists such as tamoxifen, goserelin, leuprorelin and flutamide; aromatase inhibitors such as anastrozole, fadrozole, retrozole and exemestan; immunomodulators such as aurothiomalate, D-penicillamine, bucillamine and thalidomide; immunosuppresants such as azathioprine, mizoribine, ciclosporin and rapamycin; steroidal antiinflammatory agents such as hydrocortisone, prednisolone and dexamethasone; non-steroidal antiinflammatory agents such as aspirin, indometacin, celecoxib; antihistaminic agents such as chlorpheniramine and clemastine; differentiation inducers such as tretinoin, bexarotene and arsenic; proteosome inhibitors such as bortezomib; tyrosine kinase inhibitors such as gefitinib (EGFR inhibitor), erlotinib (EGFR inhibitor), imatinib (Abl inhibitor), Flt3 inhibitor, ZD6474 (VEGFR inhibitor) and PD17034 (FGFR inhibitor); adenosine deaminase inhibitors such as pentostatin; Hsp90 inhibitors such as radicicol and 17-allylamino-17-demethoxygeldanamycin; angiogenesis inhibitors; histone deacetylase inhibitors; matrix metalloproteinase, inhibitors; farnesyl transferase inhibitors; bisphosphonate preparations; kinesin Eg5 inhibitors; serine-threonine kinase inhibitors such as UCN-01 and rapamycin; and derivatives of these compounds.

Of these agents, cisplatin, carboplatin, oxaliplatin, 5-fluorourasil, irinotecan, paclitaxel, gefitinib, melphalan, doxorubicin, bortezomib, rapamycin, Herceptin, mitoxantrone, dexamethasone, UCN-01, prednisolone and thalidomide are preferable. Melphalan, cisplatin, mitoxantrone, irinotecan, rapamycin, dexamethasone, UCN-01 and the like are more preferable.

The "medicament for treating cancer comprising a combination of a substance inhibiting activities of IGF-I and IGF-II and a substance having an antitumor activity" is an medicament in which "a substance inhibiting activities of IGF-I and IGF-II" or its pharmaceutical preparation and "a substance having an antitumor activity" or its pharmaceutical preparation are administered to an administration subject simultaneously or successively with a time difference.

That is, as a dosage form of the above agent, it is advisable that "a substance inhibiting activities of IGF-I and IGF-II" and "a substance having an antitumor activity" are combined in the administration. Examples thereof include (i) administration of a single pharmaceutical preparation obtained by simultaneously formulating "a substance inhibiting activities of IGF-I and IGF-II" and "a substance having an antitumor activity", (ii) simultaneous administration, through the same administration route, of two types of pharmaceutical preparations obtained by separately formulating "a substance inhibiting activities of IGF-I and IGF-II" and "a substance having an antitumor activity", (iii) administration, through the same administration route with a time difference, of two types of pharmaceutical preparations obtained by separately formulating "a substance inhibiting activities of IGF-I and IGF-II" and "a substance having an antitumor activity", for example, administration of "a substance inhibiting activities of IGF-I and IGF-II" and "a substance having an antitumor activity" in this order or in reverse order, (iv) simultaneous administration, through different administration routes; of two types of pharmaceutical preparations obtained by separately formulating "a substance inhibiting activities of IGF-I and IGF-II" and "a substance having an antitumor activity", (v) administration, through different administration routes with a time difference, of two types of pharmaceutical preparations obtained by separately formulating "a substance inhibiting activities of IGF-I and IGF-II" and "a substance having an antitumor activity", and the like.

When the administration is conducted with a time difference, the time difference varies depending on active ingredients to be administered, a dosage form, an administration method and the like.

The dose of the medicament for treating cancer of the present invention varies depending on the presence or absence of the irradiation used in combination, the type of the "substance having an antitumor activity" used in combination, a degree of a symptom, an administration method, an age, sex and body weight of an administration subject, a therapeutic period and the like, and it is not particularly limited. It is usually from 10 µg/kg to 10 mg/kg per day for a mammal.

Examples of diseases to which the medicament for treating cancer of the present invention is applied include various malignant and benign tumors such as malignant melanoma, malignant lymphoma, digestive cancers, lung cancer, esophageal cancer, stomach cancer, large bowel cancer, rectum cancer, colon cancer, ureteral tumor, gallbladder cancer, bile duct cancer, biliary tract cancer, breast cancer, liver cancer, pancreas cancer, testicular tumor, maxillary cancer, lingual cancer, lip cancer, mouth cancer, pharyngeal cancer, ovarian cancer, uterine cancer, prostate cancer, thyroid gland cancer, brain tumor, Kaposi's sarcoma, hemangioma, leukemia, polycythemia vera, neuroblastoma, retinoblastoma, myeloma, bladder tumor, sarcoma, osteosarcoma, myosarcoma, skin cancer, renal cancer, urinary cancer, childhood cancers, glioma and the like.

The medicament for treating cancer of the present invention is especially effective for tumor suppression of "hIGF-dependent growth cancers". The "hIGF-dependent growth cancers" refer to cancers grown in the presence of hIGF in which the degree of growth is increased dependently on the hIGF concentration. Examples thereof include prostate cancer, large bowel cancer, breast cancer, osteosarcoma, myeloma and the like.

The medicament for treating cancer of the present invention may be a medicament comprising a substance inhibiting activities of IGF-I and IGF-II and a substance having an antitumor activity solely, as an active ingredient, or a medicament comprising the both substances in combination as an active ingredient, but, usually, it is preferred to be mixed with one or more pharmaceutically acceptable carriers and is provided as a pharmaceutical composition which is manufactured by any method well known in the technical field of pharmaceutics preparations. Preferably, an aseptic solution where it is dissolved in an aqueous carrier such as water, and an aqueous solution of salt, glycine, glucose or human albumin are used. It is also possible to add a pharmaceutically acceptable additive such as buffer or isotonizing agent for making the preparation solution more similar to the physiological conditions and examples thereof are sodium acetate, sodium chloride, sodium lactate, potassium chloride and sodium citrate. It may also be preserved by freeze-drying and, in actual use, it may be used by dissolving in an appropriate solvent.

With regard to the administration route of the medicament for treating cancer of the present invention, it is preferred to use the most effective route for the treatment. Examples thereof are oral administration and parenteral administration such as intraoral, tracheobronchial, intrarectal, subcutaneous, intramuscular, intraarticular and intravenous, and, among them, intravenous administration is preferred.

Examples of the preparation suitable for the oral administration are emulsion, syrup, capsule, tablet, diluted powder and granule. Liquid preparation such as emulsion and syrup can be prepared using water, saccharides such as sucrose, sorbitol and fructose, glycols such as polyethylene glycol and propylene glycol, oils such as sesame oil, olive oil and soybean oil, antiseptics such as p-hydroxybenzoate, flavors such as strawberry flavor and peppermint flavor and the like as additives. Capsule, tablet, diluted powder, granule, and the like can be prepared using excipients such as lactose, glucose, sucrose and mannitol, disintegrating agents such as starch and sodium alginate, lubricants such as magnesium stearate and talc, binders such as polyvinyl alcohol, hydroxypropyl cellulose and gelatin, surfactants such as fatty acid ester, plasticizers such as glycerol, as additives.

Examples of the preparation suitable for parenteral administration are injection, suppository and air spray. For example, injection is prepared using a carrier comprising salt solution, glucose solution or a mixture of both, or the like. Suppository is prepared using a carrier such as cacao butter, hydrogenated fat or carboxylic acid. Air spray is prepared using the inhibiting substance as such or using, for example, a carrier which does not stimulate the mouth and the airway mucous membrane of a person to be administered, and which disperses the inhibiting substance into fine particles and makes the absorption easy. Specific examples of the carrier are lactose and glycerol. Depending upon the property of the inhibiting substance and the carrier used, it is possible to prepare aerosol, dry powder, and the like. In addition, even in the parenteral preparation, components exemplified as additives in the oral preparation may be added.

The present invention also provides a method for treating cancer which comprises administering to a mammal an effective amount of a substance inhibiting activities of IGF-I and IGF-II and an effective amount of a substance having an antitumor activity in combination, and a method for treating cancer which comprises administering to a mammal an effective amount of a substance inhibiting activities of IGF-I and IGF-II and an effective amount of a substance having an antitumor activity simultaneously or successively.

The "mammal" here refers to a mammal having a cancer, and it includes humans, dogs, cats, sheep, goat, cattle, horses, pigs and the like. The "effective amount" refers to an amount which stops growth of cancer cells, reduces a tumor size or allows a tumor to disappear by the administration of the substances to cancer cells during growth.
II. Preparation of antibody or antibody fragment used in the medicament for treating cancer of the present invention

Hereunder, a process for producing the antibody or the antibody fragment being one of the substances used in the present invention which specifically binds to IGF-I and IGF-II and inhibits the activity of IGF-I and IGF-II, and evaluation of activity thereof will be mentioned.

### 1. Preparation of Monoclonal antibody-producing hybridoma to IGF

### (1) Preparation of antigen

Expression vector comprising cDNA encoding for IGF is introduced and expressed in *Escherichia coli,* yeast, insect cell, animal cell, and the like to thereby obtain recombinant IGF protein and the resulting protein can be used as an antigen. Alternatively, a synthetic peptide having an IGF partial sequence can also be used as an antigen.

With regard to a partial peptide for antigen, a partial protein sequence of about 5 to 30 residues is selected. In order to obtain an antibody which recognizes the protein in a state of having a non-denatured natural structure, it is necessary to select a partial sequence existing on the surface of protein in view of three-dimensional structure as an antigen peptide. The part existing on the surface of protein in view of three-dimensional structure can be presumed by predicting a highly hydrophilic partial sequence using commercially available software for analysis of protein sequence such as Genetyx Mac. Thus, that is because, in general, there are many cases where a lowly hydrophilic region is present in the inner part of the protein in view of three-dimensional structure and there are many cases where a highly hydrophilic region is present on the surface of protein. In addition, there are many cases where N-terminal and C-terminal of protein are present on the surface of protein. However, the partial peptide which is selected as such will not always be an antigen which establishes the desired antibody.

In order to cross-link to protein, cysteine is added to the terminal of a partial peptide. When an internal sequence of protein is selected as a partial peptide, N-terminal and C-terminal of the peptide are acetylated and amidated, respectively, if necessary. A partial peptide can be synthesized by a common liquid-phase or solid-phase peptide synthetic method, a method where they are appropriately combined or a modif ied method thereof (The Peptides, Analysis, Synthesis, Biology, Vol. 1, 1979; Vol. 2, 1980; Vol. 3, 1981, Academic Press; Fundamentals and Experiments for Peptide Synthesis, Maruzen, 1985; Development of Drugs, Second Series, Vol. 14, Peptide Synthesis, Hirokawa Shoten, 1991; *International Journal of Protein* & *Protein Research,* 35, 161-214, 1990). It is also possible to use an automated peptide synthesizer. Synthesis of peptide using a peptide synthesizer can be carried out on a commercially available peptide synthesizer such as a peptide synthesizer manufactured by Shimadzu, a peptide synthesizer manufactured by Applied Biosystems, Inc. (hereinafter, referred to as ABI) and a peptide synthesizer manufactured by Advanced ChemTech Inc. (hereinafter, referred to as ACT) using Nα-Fmoc-amino acid or Nα-Boc-amino acid where side chain is appropriately protected according to synthetic program for each of them.

Protected amino acids used as raw material and carrier resins are available from ABI, Shimadzu, Kokusan Kagaku, Nova Biochem, Watanabe Kagaku, ACT, Peptide Laboratory, etc. Protected amino acids, protected organic acids and protected organic amines used as starting materials may also be synthesized by already-reported synthetic methods or modified methods thereof (The Peptides, Analysis, Synthesis, Biology, Vol. 1, 1979; Vol. 2, 1980; Vol. 3, 1981, Academic Press; Fundamentals and Experiments for Peptide Synthesis, Maruzen, 1985; Development of Drugs, Second Series, Vol. 14, Peptide Synthesis, Hirokawa Shoten, 1991; *International Journal of Protein & Protein Research,* 35, 161-214 , 1990).

### (2) Immunization of animal and, preparation of antibody-producing cell

With regard to the animal used for immunization, any animals may be used so long as it can prepare hybridoma such as mouse, rat, hamster and rabbit. Hereunder, examples using mouse and rat will be illustrated.

3 to 20-weeks old mice or rats were immunized with the antigen which was prepared in aforementioned 1(1) and antibody-producing cells were collected from spleen, lymph node and peripheral blood of the animal. Immunization is carried out by administrating antigen to the animal for several times together with an appropriate adjuvant either subcutaneously, intravenously or intraperitoneally. Examples of the adjuvant are complete Freund's adjuvant or aluminum hydroxide gel, pertussis vaccine and the like. A complex is prepared with carrier protein such as bovine serum albumin (hereinafter, referred to as BSA) or keyhole limpet hemocyanin (hereinafter, referred to as KLH) and the resulting complex can be used as an immunogen. After 3 to 7 days from administrating each antigen, blood is collected from venous plexus of fundus of the eye or tail vein of the immunized animal, its reactivity to hIGF used as an antigen is confirmed by means of ELISA or the like and the mouse or rat where its serum shows a sufficient antibody value is used as a source for antibody-producing cell. On the 3 to 7 days from the final administration of the antigen, spleen, etc. are excised from the immunized mouse or rat according to a known method (Antibodies - A Laboratory Manual, Cold Spring Harbor Laboratory, 1988) and antibody-producing cells and myeloma cells are fused.

### (3) Preparation of myeloma cell

With regard to the myeloma cell, any myeloma cell may be used so long as it can proliferate *in* vitro such as 8-azaguanine-resistant myeloma cell line P3-X63Ag8-U1 (P3-U1) (*European Journal of Immunology,* 6, 511-519, 1976), SP2/0-Ag14 (SP-2) (Nature, 276, 269-270, 1978), P3-X63-Ag8653 (653) (*Journal of Immunology,* 123, 1548-1550, 1979), P3-X63-Ag8 (X63) (Nature, 256, 495-497, 1975) or the like. The culturing and sub-culturing of those cell lines can be carried out in accordance with a known method (Antibodies - A Laboratory Manual, Cold Spring Harbor Laboratory, 1988) to thereby secure not less than 2 × 10⁷ cells until the stage of cell fusion.

### (4) Cell fusion

The antibody-producing cell and myeloma cell prepared hereinabove are washed and a cell-aggregating medium such as polyethylene glycol-1000 (hereinafter, referred to as PEG-1000) is added thereto whereupon cells are fused and suspended in a medium. For washing the cells, modified Eagle' s medium (hereinafter, referred to as MEM), phosphate buffered saline (hereinafter, referred to as PBS) or the like is used. With regard to a medium in which the fused cells are suspended, an HAT medium {a medium where 0.1 mM hypoxanthine, 15 µM thymidine and 0.4 µM aminopterin are added to a common medium [a medium where 1.5 mM glutamine, 50 µM 2-mercaptoethanol, 10 µg/mL gentamicin and 10% fetal bovine serum (hereinafter, referred to as FBS) are added to an RPMI-1640 medium]} is used so that the desired fused cell is selectively obtained.

After the culturing, a part of the culture supernatant liquid is taken out and a sample which reacts with antigen protein and does not react with non-antigen protein is selected by ELISA. After that, a limiting diluting method is carried out to make it into a single cell and a sample which showed a stable and high antibody titer by ELISA is selected as a monoclonal antibody-producing hybridoma.

### (5) Selection of hybridoma

Selection of hybridoma which produces an anti-hIGF monoclonal antibody is carried out by ELISA which will be mentioned later in accordance with a known method (Antibodies - A Laboratory Manual, Cold Spring Harbor Laboratory, 1988). According to such a method, it is now possible to measure a binding activity of antibody contained in a culture supernatant of transformant cell line which produces anti-hIGF chimeric antibody, anti-hIGF CDR-grafted antibody or antibody fragment thereof which will be mentioned later or all pure antibodies.

### ELISA

Antigen is fixed in a 96-well ELISA plate and reaction is carried out using a culture supernatant of such as hybridoma or purified antibody as the first antibody. After the reaction of the first antibody, the plate is washed and the second antibody is added. With regard to the second antibody, an antibody which can recognize the first antibody is labeled with biotin, enzyme, chemiluminescent substance, radioisotope or the like is used. To be more specific, when mouse is used in the preparation of the hybridoma, an antibody which can recognize the mouse antibody is used as the second antibody. After the reaction, the reaction corresponding to the labeled substance of the second antibody is carried out to select a hybridoma producing a monoclonal antibody which specifically reacts with the antigen.

A specific example of the hybridoma is a hybridoma KM 1468. The hybridoma KM 1468 has been deposited as FERM BP-7978 on March 26, 2002 under the stipulation of the Budapest Treaty at the International Patent Organism Depositary of the National Institute of Advanced Industrial Science and Technology (Central No. 6, 1-1, Higashi-1-chome, Tsukuba-Shi, Ibaragi-Ken, Postal Code; 305-8566).

### (6) Purification of monoclonal antibody

Anti-hIGF monoclonal antibody-producing hybridoma cell obtained in 1(4) is intraperitoneally injected in an amount of 5 × 10⁶ to 2 × 10⁷ cells/mouse to a mouse or nude mouse of 8 to 10-weeks old to which 0.5 mL of pristane (2,6,10,14-tetramethylpentadecane) is intraperitoneally administered followed by breeding for two weeks. With 10 to 21 days, the hybridoma becomes ascites tumor. The ascites is collected from the mouse or nude mouse, centrifuged, salting out with 40 to 50% saturated ammonium sulfate, subjected to a precipitation method with caprylic acid and IgG or IgM fraction is recovered by using DEAE-Sepharose column, protein A column, column of Cellulofine GSL 2000 (manufactured by Seikagaku Kogyo) or the like to prepare purified monoclonal antibody.

The subclass of the purified monoclonal antibody can be determined by using a mouse monoclonal antibody typing kit, a rat monoclonal antibody typing kit or the like. Concentration of protein can be calculated by a Lowry method or from the absorbance at 280 nm.

The subclass of the antibody means an isotype in the class and includes IgG1, IgG2a, IgG2b and IgG3 in the case of mouse and IgG1, IgG2, IgG3 and IgG4 in the case of human.

### (7) Activity evaluation of monoclonal antibody

### (7-1) Evaluation of binding activity to hIGF

Binding activity of the anti-hIGF monoclonal antibody which is in a culture supernatant or is purified can be measured by ELISA in aforementioned 1(5), surface plasmon resonance *(Journal of Immunological Methods,* 145, 229-240, 1991), and the like. Reactivity with hIGF and antigen epitope can also be analyzed by a competitive ELISA using hIGF and partial peptides of hIGF. It can be presumed by a commonly conducted three-dimensional structural analytical method or combination of various immunological methods whether the antibody recognizes the three-dimensional structure of hIGF. Examples of the three-dimensional structural analytical method are X-ray crystallography and nuclear magnetic resonance method. An example of a combination of various immunological methods is a combination of ELISA to non-denatured antigen with ELISA to denatured antigen. In that case, the antibody shows reactivity only to non-denatured antigen, it is highly presumed that it recognizes the three-dimensional structure of the antigen. An example of ELISA to non-denatured antigen is ELISA where non-denatured antigen is allowed to react with antibody in a liquid phase. With regard to ELISA to denatured antigen, any method may be used so long as it is ELISA in which antibody is made to react under such a state that antigen does not have its natural three-dimensional structure and its examples are ELISA to antigen which is directly fixed on a hydrophobic reaction plate and to partial peptide which is digested into an appropriate length.

The antibody used in the present invention can be obtained by selecting an antibody having a binding activity to hIGF-II and a binding activity to hIGF-I by the measuring method for binding activity or by a competitive ELISA.

By examining the influence to cell line showing an hIGF-dependent proliferation, the inhibitory activity of the activity of hIGF *in vitro* can also be measured. Examples of cell line showing an hIGF-I- or hIGF-II-dependent proliferation are human breast cancer cell strain MCF7 (ATCC HTB-22), human colorectal cancer cell strain HT-29 (ATCC HTB-38) and the like.

Further, by establishing an hIGF-dependent cell proliferation measuring system using animal such as mice and examining influence on the measuring system, activity which inhibits the activity of hIGF *in vivo* can be measured.

### 2. Preparation of polyclonal antibody of non-human animal to IGF

Polyclonal antibody can be prepared from serum of an animal where its serum shows a sufficient antibody titer among the animal to which immune is applied by the above method mentioned in 1.(2).

Thus, the serum fractionated by a centrifugation from the blood recovered from the animal or the immunoglobulin fraction is purified from the serum by a conventional method whereupon the polyclonal antibody can be prepared. With regard to activity of the polyclonal antibody, a binding activity to antigen can be evaluated by the above method mentioned in 1.(7).

### 3. Preparation of humanized antibody

### (1) Construction of vector for expression of humanized antibody

With regard to a vector for expression of humanized antibody, any vector for expression of humanized antibody may be used so long as it is vector for expression in animal cell into which gene coding for CH and/or CL of human antibody is inserted. Vector for expression of humanized antibody can be constructed by cloning the genes coding for CH and CL of human antibody, respectively, into vector for expression in animal cell.

C region of human antibody may be CH and CL of any human antibody and its examples are C region of IgG1 subclass of H chain of human antibody (hereinafter, referred to as hCγ1) and C region of κ class of L chain of human antibody (hereinafter, referred to as hCκ). With regard to the genes coding for CH and CL of human antibody, a chromosome DNA comprising exon and intron may be used and also, cDNA may be used.

With regard to vector for expression in animal cell, any vector may be used so long as the gene coding for the C region of human antibody can be inserted and expressed. Its examples are pAGE107 (*Cytotechnology*, 3, 133-140, 1990), pAGE103 (*Journal of Biochemistry*, 101, 1307-1310, 1987), pHSG274 (*Gene*, 27, 223-232, 1984), pKCR (*Proceedings of the National Academy of Sciences of the United State of America,* 78, 1527-1531, 1981) and pSG1βd2-4 (*Cytotechnology, 4,* 173-180, 1990). Examples of promoter and enhancer used for the expression vector for animal cells are SV40 initial promoter and enhancer *(Journal of Biochemistry,* 101, 1307-1310, 1987), Moloney mouse leukemia virus *LTR* promoter and enhancer *(Biochemical* & *Biophysical Research Communications,* 149, 960-968, 1987) and immunoglobulin H chain promoter *(Cell,* 41, 479-487, 1985) and enhancer *(Cell,* 33, 717-728, 1983).

With regard to the vector for expression of human antibody, either of a type where antibody H chain and L chain are on different vectors and where they are on the same vector (hereinafter, referred to as a tandem type) may be used but, in view of easiness of construction of humanized antibody expression vector, easiness of introduction into animal cells and well-balanced expressed amount of antibody H chain and L chain in animal cells, a tandem type of vector for expression of humanized antibody is preferred *(Journal of Immunological Methods,* 167, 271-278, 1994). Examples of a tandem type of vector for expression of humanized antibody are pKANTEX93 (WO 97/10354) and pEE18 *(Hybridoma*, 17, 559-567, 1998).

The constructed vector for expression of humanized antibody can be used for expression of human chimeric antibody and human CDR-grafted antibody in animal cell.

### (2) Obtaining of cDNA coding for V region of antibody of non-human animal and analysis of amino acid sequence thereof

cDNA which codes for antibody of non-human animal such as VH and VL of mouse antibody is obtained as follows.

mRNA is extracted from hybridoma which produces mouse antibody, etc. and cDNA is synthesized. The synthesized cDNA is cloned into vector such as plasmid or phage to prepare a cDNA library. Using C region or V region of the mouse antibody as a probe, each of recombinant phage or recombinant plasmid having cDNA coding for VH or recombinant phage or recombinant plasmid having cDNA coding for VL is isolated from the library. Full length of nucleotide sequences of VH and VL of the desired mouse antibody on the recombinant phage or recombinant plasmid are determined and the full length of the amino acid sequences of VH and VL are deduced from the nucleotide sequences.

With regard to non-human animal, any animal such as mouse, rat, hamster and rabbit may be used so long as it can prepare a hybridoma.

An example of a method for preparing the total RNA from hybridoma is a guanidine thiocyanate-cesium trifluoroacetate method *(Methods in Enzymology,* 154, 3-28, 1987) and an example of a method for preparing mRNA from the total RNA is an oligo (dT) immobilized cellulose column method (Molecular Cloning: A Laboratory Manual, Cold Spring Harbor Lab. Press, New York, 1989). Examples of a kit for the preparing mRNA from hybridoma are Fast Track mRNA Isolation Kit (manufactured by Invitrogen), Quick Prep mRNA Purification Kit (manufactured by Pharmacia), and the like.

Examples of a method for the synthesizing of cDNA and for preparing cDNA library are a conventional method (Molecular Cloning: A Laboratory Manual, Cold Spring Harbor Lab. Press, New York, 1989; Current Protocols in Molecular Biology, Supplement 1-34) and a method using a commercially available kit such as Super Script™ Plasmid System for cDNA Synthesis and Plasmid Cloning (manufacture by Gibco BRL) and ZAP-cDNA Synthesis Kit (manufactured by Stratagene).

With regard to vector into which cDNA synthesized using mRNA extracted from the hybridoma as a template is inserted while preparing the cDNA library, any vector may be used so long as the cDNA can be inserted therein. For example, phage or plasmid vector such as ZAP Express *(Strategies,* 5, 58-61, 1992), pBluescript II SK(+) *(Nucleic Acid Research,* 17, 9494, 1989), λ ZAP II (manufactured by Stratagene), λ, gt 10 and λ gt 11 (DNA Cloning: A Practical Approach, I, 49, 1985), Lambda BlueMid (manufactured by Clontech), λ, ExCell, pT7T3 18U (manufactured by Pharmacia), pcD2 *(Molecular* & *Cellular Biology,* 3, 280-289, 1983) and pUC 18 *(Gene,* 33, 103-119, 1985) may be used.

With regard to *Escherichia coli* into which a cDNA library constructed by phage or plasmid vector is introduced, any *Escherichia coli* may be used so long as it the cDNA library can be inserted, expressed and maintained. Its examples are XL1-Blue MRF' (*Journal of Biotechnology,* 23, 271-289, 1992), C600 (*Genetics,* 59, 177-190, 1968), Y1088 and Y1090 (*Science,* 222, 778-782, 1983), NM 522 (*Journal of Molecular Biology,* 166, 1-19, 1983), K 802 (*Journal of Molecular Biology,* 16, 118-133, 1966), JM 105 (*Gene,* 38, 275-276, 1985) and the like.

With regard to a method for selecting cDNA clones coding for VH and VL of antibody of non-human animal from cDNA library, it can be selected by a colony hybridization method or a plaque hybridization method (Molecular Cloning: A Laboratory Manual, Cold Spring Harbor Lab. Press, New York, 1989) using radioisotope or fluorescence-labeled probe. In addition, cDNAs coding for VH and VL can be prepared by a polymerase chain reaction (hereinafter, referred to as PCR method; Molecular Cloning: A Laboratory Manual, Cold Spring Harbor Lab. Press, New York, 1989; Current Protocols in Molecular Biology, Supplement 1-34) by preparing primer and cDNA synthesized from mRNA or cDNA library as a template.

cDNA selected by the above-mentioned method is cleaved by an appropriate restriction enzyme or the like, cloned to a plasmid vector such as pBluescript SK(-) (manufactured by Stratagene), subjected to a method conventionally used for analysis of nucleotide sequence such as a dideoxy method *(Proceedings of the National Academy of Sciences of the United States of America,* 74, 5463-5467, 1977) and analyzed by an automatic sequencer (ABI PRISM 377 (manufactured by ABI)) or the like whereupon a nucleotide sequence of the cDNA can be determined.

The full length of amino acid sequences of VH and VL are deduced from the determined nucleotide sequences and compared with the full length of amino acid sequences of VH and VL of known antibody (Sequences of Proteins of Immunological Interest, U. S. Dept. Health and Human Services, 1991) whereupon it can be confirmed whether the obtained cDNA codes for the full length of amino acid sequences of VH or VL of the antibody containing a signal sequence for secretion. With regard to the full length of amino acid sequences of VH or VL of the antibody containing a signal sequence for secretion, length and N-terminal amino acid sequence of the signal sequence can be deduced by comparing with the full length of amino acid sequences of VH and VL of the known antibody (Sequences of Proteins of Immunological Interest, U. S. Dept. Health and Human Services, 1991) and, further, subclass to which they belong can be determined. Also an amino acid sequence of each CDR of VH and VL can be found by comparing with the amino acid sequences of VH and VL of the known antibody (Sequences of Proteins of Immunological Interest, U. S. Dept. Health and Human Services, 1991).

A homology search of sequences such as a BLAST method *(Journal of Molecular Biology,* 215, 403-410, 1990) to any database such as SWISS-PROT or PIR-Protein can be conducted using the full length of amino acid sequences of VH and VL to examine novelty of the sequence.

### (3) Construction of human chimeric antibody expression vector

cDNAs coding for VH and VL of antibody of non-human animal are cloned in the upstream of genes coding for CH and CL of human antibody of vector for expression of humanized antibody mentioned in the above 2(1) to thereby construct human chimeric antibody expression vector. For example, each cDNA coding for VH and VL of antibody of non-human animal is ligated to synthetic DNA comprising a nucleotide sequence of 3' -terminal of VH and VL of antibody of non-human animal and a nucleotide sequence of 5'-terminal of CH and CL of human antibody and having recognition sequence of an appropriate restriction enzyme at both ends, and cloned so that each of them is expressed in an appropriate form in the upstream of gene coding for CH and CL of human antibody of the vector for expression of humanized antibody mentioned in the above 2(1) to construct human chimeric antibody expression vector. In addition, cDNA coding for VH and VL is amplified by a PCR method using a primer having a recognition sequence of an appropriate restriction enzyme at 5'-terminal using a plasmid containing cDNA coding for VH and VL of antibody of non-human animal and each of them is cloned so that it is expressed in an appropriate form in the upstream of gene coding for CH and CL of human antibody of the vector for expression of humanized antibody mentioned in the above 2(1) to construct human chimeric antibody expression vector.

### (4) Construction of cDNA coding for V region of human CDR-grafted antibody

cDNAs coding for VH and VL of human CDR-grafted antibody can be constructed as follows. Firstly, amino acid sequence of FRs in VH and VL of human antibody to which the desired amino acid sequences of CDRs in VH and VL of non-human animal is selected. With regard to the amino acid sequence of FRs in VH and VL of human antibody, any amino acid sequence of FRs in VH and VL of human antibody may be used so long as it is derived from human antibody. Examples thereof are amino acid sequences of FRs in VH and VL of human antibody registered in database such as Protein Data Bank and a consensus amino acid sequence of each subgroup of FRs in VH and VL of human antibody (Sequences of Proteins of Immunological Interest, U. S. Dept. Health and Human Services, 1991). In order to prepare a human CDR-grafted antibody having a sufficient activity, an amino acid sequence having a homology of as high as possible (60% or more) to the amino acid sequence of FRs in VH and VL of antibody of the desired non-human animal among the above is preferably selected. After that, the amino acid sequence of CDRs in VH and VL of the desired non-human animal antibody is grafted to the selected amino acid sequence of FRs in VH and VL of the human antibody to design the amino acid sequences of VH and VL of the human CDR-grafted antibody. The designed amino acid sequences are converted to nucleotide sequences by considering the frequency of codon usage (Sequences of Proteins of Immunological Interest, U. S. Dept. Health and Human Services, 1991) found in the nucleotide sequence of gene of antibody whereupon nucleotide sequences coding for amino acid sequences of VH and VL of the human CDR-grafted antibody are designed. Based on the designed nucleotide sequences, several synthetic DNAs having a length of about 100 bases are synthesized and a PCR method is carried out by using them. In this case, it is preferred to design six synthetic DNAs for both VH and VL in view of reaction efficiency in the PCR and length of synthesizable DNA.

Further, by introducing a recognition sequence of an appropriate restriction enzyme into 5'-terminal of synthetic DNAs located at both ends, cloning to a vector for expression of humanized antibody constructed in the above 2(1) can be carried out. After the PCR, the amplified product is cloned to a plasmid such as pBluescript SK(-) (manufactured by Stratagene) and a nucleotide sequence is determined by the method mentioned in the above 2(2) whereupon a plasmid having nucleotide sequences coding for the amino acid sequences of VH and VL of the desired human CDR-grafted antibody is obtained. (5) Modification of amino acid sequence of V region of human CDR-garafted antibody

It has been known that, when a human CDR-garafted antibody is produced by simply grafting the CDRs in VH and VL of an desired antibody of the non-human animal into FRs in VH and VL of human antibody, antigen-binding activity of human CDR-grafted antibody is lower as compared with the original antibody of the non-human animal *(Bio*/*Technology,* 9, 266-271, 1991). With regard to the cause thereof, it is considered that, in the original VH and VL of antibody of the non-human animal, not only CDRs but also some amino acid residues of FRs participate in antigen-binding activity either directly or indirectly and that, as a result of grafting of CDRs, such amino acid residues change to amino acid residues being different from FRs in VH and VL of the human antibody. In order to solve the problem, it has been conducted in a human CDR-grafted antibody that, among the amino acid sequence of FRs in VH and VL of human antibody, an amino acid residue which directly relates to binding to the antigen, or amino acid residue which indirectly relates to binding to an antigen by interacting with an amino acid residue in CDRs or by maintaining the three-dimensional structure of an antibody, is identified and that the amino acid residues is modified to amino acid residues found in the original antibody of non-human animal to thereby increase the lowered antigen-binding activity (*Bio*/*Technology*, 9, 266-271, 1991). In the preparation of human CDR-grafted antibody, how to efficiently identify the amino acid residues of relating to the antigen binding activity in FR is most important, so that the three dimensional structure of an antibody is constructed and analyzed by X-ray crystallography (*Journal of Molecular Biology,* 112, 535-542, 1977), a computer-modeling (*Protein Engineering*, 7, 1501-1507, 1994), or the like. Information for three-dimensional structure of the antibody as such has given much advantageous information to the preparation of human CDR-grafted antibody but, on the other hand, no method for the preparing of human CDR-grafted antibody which is applicable to any antibodies has not been established yet and, at present, various trials and errors are necessary such as that several kinds of modified products are prepared for each antibody and that correlation to each antigen binding activity is examined.

Modification of amino acid residue of FRs in VH and VL of human antibody can be achieved by conducting a PCR method mentioned in the above 2(4) using a synthetic DNA for the modification. With regard to the amplified product after the PCR, its nucleotide sequence is determined by the method mentioned in the above 2(2) whereby it is confirmed that the desired modification has been done.

### (6) Construction of the human CDR-grafted antibody expression vector

cDNAs coding for VH and VL of the human CDR-grafted antibody constructed in the above 2(4) and (5) are cloned to the upstream of genes coding for CH and CL of the human antibody in the vector for expression of the humanized antibody mentioned in the above 2(1) to thereby construct a human CDR-grafted antibody expression vector. For example, in the synthetic DNA used for the construction of VH and VL of the human CDR-grafted antibody in the above 2(4) and (5), recognition sequences of an appropriate restriction enzyme are introduced into 5'-terminal of the synthetic DNAs located at both ends whereby they can be cloned to the upstream of genes coding for CH and CL of human antibody in the vector for expression of humanized antibody mentioned in the above 2(1) in such a manner that they are expressed in an appropriate form.

### (7) A transient expression of humanized antibody

In order to efficiently evaluate the antigen-binding activity of the various humanized antibodies prepared, a transient expression of humanized antibody can be conducted using the humanized antibody expression vector mentioned in the above 2 (3) and (6) or the modified expression vector thereof. With regard to a host cell into which the expression vector is introduced, any cell may be used so long as it is a host cell which can express the humanized antibody, and COS-7 cell (ATCC CRL-1651) has been commonly used in view of its high expressing amount (Methods in Nucleic Acids Research, CRC Press, 283, 1991). The methods for the introducing the expression vector into COS-7 cells are DEAE-dextran method (Methods in Nucleic Acids Research, CRC Press, 283, 1991), a lipofection method *(Proceedings of the National Academy of Science of the United States of America,* 84, 7413-7417, 1987), and the like. After introducing the expression vector, the amount of humanized antibody expressed in the culture supernatant and antigen-binding activity can be measured by, for example, ELISA (Antibodies: A Laboratory Manual, Cold Spring Harbor Laboratory, Chapter 14, 1988; Monoclonal Antibodies: Principles and Practice, Academic Press Limited, 1996).

### (8) Stable expression of humanized antibody

A transformant cell which stably expresses humanized antibody can be obtained by introducing the humanized antibody expression vector mentioned in the above 2(3) and (6) into appropriate host cell. The methods for the introducing expression vector into host cell are an electroporation method *(Cytotechnology,* 3, 133-140, 1990), and the like. With regard to the host cell into which humanized antibody expression vector is introduced, any cell may be used so long as it is a host cell which can express the humanized antibody. Examples thereof are mouse SP2/0-Ag14 cell (ATCC CRL-1581), mouse P3X63-Ag8.653 cell (ATCC CRL-1580), dihydrofolate reductase gene (hereinafter, referred to as *dhfr*)-deficient CHO cell *(Proceedings of the National Academy of Sciences of the United States of America,* 77, 4216-4220, 1980) and rat YB2/3HL.P2.G11.16Ag.20 cell (ATCC CRL-1662; hereinafter, referred to as YB2/0 cell).

A transformant in which humanized antibody is stably expressed after introducing expression vector can be selected by culturing in a medium for animal cell culture containing an agent such as G418 sulfate (hereinafter, referred to as G418) according to a process disclosed in Japanese Published Unexamined Patent Application NO. 257891/90. With regard to a medium for culturing animal cell, RPMI 1640 medium (manufactured by Nissui Seiyaku), GIT medium (manufactured by Nippon Seiyaku), EX-CELL 302 medium (manufactured by JRH), IMDM (manufactured by Gibco BRL), Hybridoma-SFM (manufactured by Gibco BRL), a medium obtained by adding various additives such as FBS thereto, and the like may be used. When the resulting transformant cell is cultured in a medium, humanized antibody can be expressed and accumulated in the culture supernatant. The amount of the humanized antibody expressed in the culture supernatant and antigen-binding activity can be measured by ELISA. Further, in the transformant cell, the amount of the humanized antibody expressed can be increased by utilizing a *dhfr* system or the like according to a method disclosed in Japanese Published Unexamined Patent Application NO. 257891/90.

Humanized antibody can be purified from the culture supernatant of the transformant cell using a protein A column (Antibodies: A Laboratory Manual, Cold Spring Harbor Laboratory, Chapter : 8, 1988; Monoclonal Antibodies: Principles and Practice, Academic Press Limited, 1996). Besides that, purifying methods which are usually used for purification of proteins can be used. For example, gel filtration, ion-exchange chromatography and ultrafiltration may be conducted in combination so as to purify. Molecular weight of H chain and L chain of the purified humanized antibody or of the whole antibody molecular can be determined by a polyacrylamide gel electrophoresis (hereinafter, referred to as PAGE; *Nature,* 227, 680-685, 1970), a western blotting method (Antibodies: A Laboratory Manual, Cold Spring Harbor Laboratory, Chapter 12, 1988; Monoclonal Antibodies: Principles and Practice, Academic Press Limited, 1996), and the like.

### (9) Evaluation of activity of humanized antibody

Evaluation of activity of humanized antibody can be carried out in the same manner as in the above 1(7).

### 4. Preparation of antibody fragment

Antibody fragment can be prepared from the anti-hIGF antibody mentioned in the above 1 and 2 by genetic engineering techniques or protein chemical techniques.

Examples of the genetic engineering techniques are a method where gene coding for desired antibody fragment is constructed and expression and purification are conducted using a suitable host such as animal cells, plant cells, insect cells, *Escherichia coli,* or the like.

Examples of the protein chemical techniques are a method of site-specific cleavage, purification using a protease such as pepsin and papain, and the like.

A process for producing of Fab, F(ab')₂, Fab' , scFv, diabody, dsFv or CDR-containing peptide as a antibody fragment will be specifically illustrated as follows.

### (1) Preparation of Fab

Fab can be prepared by treating IgG with protease, papain by using protein chemical techniques. After the treatment with papain, it is possible to recover as a uniform Fab by passing through a protein A column to separate from IgG molecule and Fc fragment provided that the original antibody is an IgG subclass having a binding property to protein A (Monoclonal Antibodies: Principles and Practice, third edition, 1995). In the case of an antibody of an IgG subclass having no binding property to protein A, Fab can be recovered by an ion-exchange chromatography at a fraction eluted by a low salt concentration (Monoclonal Antibodies: Principles and Practice, third edition, 1995). Fab can also be prepared by genetic engineering techniques using *E. coli* in many cases or using insect cells, animal cells, and the like. For example, DNA coding for V region of the antibody mentioned in the above 2(2), 2(4) and (5) is cloned to a vector for expression of Fab whereupon Fab expression vector can be prepared. With regard to vector for expression of Fab, any vector may be used so long as DNA for Fab can be inserted and expressed. An example thereof is pIT 106 (*Science,* 240, 1041-1043, 1988). Fab expression vector is introduced into an appropriate *E. coli* whereby Fab can be formed and accumulated in an inclusion body or a periplasmic space. From the inclusion body, active Fab can be obtained by a refolding method generally used for proteins and, when expressed in periplasmic space, active Fab leaks out in a culture supernatant. After the refolding or from the culture supernatant, a uniform Fab can be purified using a column to which antigen is bound (Antibody Engineering, A Practical Guide, W. H. Freeman and Company, 1992).

### (2) Preparation of F(ab')₂

F(ab')₂ can be prepared by treating of IgG with protease, pepsin by using protein chemical techniques. After the treatment with pepsin, it can be recovered as a uniform F(ab')₂ by the same purifying operation as in the case of Fab (Monoclonal Antibodies: Principles and Practice, third edition, Academic Press, 1995). It can also be prepared by a method where Fab' mentioned in the following 3(3) is treated with a maleimide such as o-PDM or bismaleimide to form a thioether bond or by a method where it is treated with DTNB [5,5'-dithiobis(2-nitrobenzoic acid)] to form an S-S bond (Antibody Engineering, A Practical Approach, IRL Press, 1996).

### (3) Preparation of Fab'

Fab' can be prepared by treating F(ab')₂ mentioned in the above 3(2) with a reducing agent such as dithiothreitol. Fab' can be prepared by genetic engineering techniques using *E*. *coli* in many cases or using insect cells, animal cells, and the like. For example, DNA coding for V region of the antibody mentioned in the above 2(2), 2(4) and 2(5) is cloned to a vector for expression of Fab' whereupon Fab' expression vector is able to be prepared. With regard to a vector for expression of Fab' , any vector may be used so long as DNA for Fab' can be inserted and expressed. An example thereof is pAK 19 *(Bio*/*Technology*, 10, 163-167, 1992). The Fab' expression vector is introduced into an appropriate *E. coli* to form and accumulate Fab' in an inclusion body or periplasmic space. From the inclusion body, active Fab' can be obtained by a refolding method which is usually used in proteins and, when the Fab' is expressed in periplasmic space, it can be recovered extracellulary by disrupting the cell with treating such as partial digestion by lysozyme, osmotic shock and sonication. After the refolding or from the disrupted cell solution, a uniform Fab' can be purified using a protein G column or the like (Antibody Engineering, A Practical Approach, IRL Press, 1996).

### (4) Preparation of scFv

scFv can be prepared using phage or *E. coli* or using insect cells or animal cells by genetic engineering techniques. For example, DNA coding for V region of the antibody mentioned in the above 2(2), 2(4) and 2(5) is cloned to a vector for expression of scFv whereupon an scFv expression vector is able to be prepared. With regard to the vector for expression of scFv, any vector may be used so long as the DNA of scFv can be inserted and expressed. Examples thereof are pCANTAB5E (manufactured by Pharmacia), pHFA *(Human Antibodies & Hybridomas*, 5, 48-56, 1994), and the like. When scFv expression vector is introduced into an appropriate *E. coli* and a helper phage is infected, a phage which expresses scFv on the phage surface in a fused form with the surface protein of the phage can be obtained. Also, scFv can be formed and accumulated in periplasmic space or an inclusion body of *E. coli* into which scFv expression vector is introduced. From the inclusion body, active scFv can be obtained by a refolding method generally used for proteins and, when scFv is expressed in periplasmic space, it can be recovered extracellulary by disrupting the cell with treating such as partial digestion by lysozyme, osmotic shock and sonication. After the refolding or from the disrupted cell solution, a uniform scFv can be purified using a cation-exchange chromatography or the like (Antibody Engineering, A Practical Approach, IRL Press, 1996).

### (5) Preparation of diabody

Diabody can be prepared using *E*. *coli* in many cases or using insect cells, animal cells, and the like by genetic engineering techniques. For example, DNAs in which VH and VL of the antibody mentioned in the above 2(2), 2(4) and 2(5) are linked by a linker coding 8 amino acid residues or less is prepared and cloned into a vector for expression of diabody whereupon a diabody expression vector can be prepared. With regard to a vector for expression of diabody, any vector may be used so long as the DNA of diabody can be inserted and expressed. Examples thereof are pCANTABSE (manufactured by Pharmacia) andpHFA *(Human Antibodies Hybridomas* , 5, 48, 1994). Diabody can be formed and accumulated in periplasmic space or inclusion body of *E. coli* into which a diabody expression vector is introduced. From the inclusion body, active diabody can be obtained by a refolding method generally used for proteins and, when the diabody is expressed in periplasmic space, it can be recovered extracellulary by disrupting the cell with treating such as partial digestion by lysozyme, osmotic shock and sonication. After the refolding or from the disrupted cell solution, a uniform scFv can be purified using a cation-exchange chromatography or the like (Antibody Engineering, A Practical Approach, IRL Press, 1996).

### (6) Preparation of dsFv

dsFv can be prepared using *E. coli*. in many cases or using insect cells, animal cells, and the like by genetic engineering techniques. Firstly, mutation is introduced into an appropriate position of DNA coding for VH and VL of the antibody mentioned in the above 2(2), 2(4) and 2(5) to prepare DNAs in which an encoded amino acid residue is replaced with cysteine. Each DNA prepared as such is cloned to a vector for expression of dsFv whereby an expression vector of VH and VL can be prepared. With regard to a vector for expression of dsFv, any vector may be used so long as the DNA for dsFv can be inserted and expressed. An example thereof is pULI 9 *(Protein Engineering*, 7, 697-704, 1994). The expression vector of VH and VL is introduced into an appropriate *E*. *coli* and dsFv is formed and accumulated in an inclusion body or periplasm. VH and VL are obtained from the inclusion body or periplasm, mixed and subjected to a refolding method generally used for proteins to thereby obtain active dsFv. After the refolding, it can be further purified by an ion-exchange chromatography, a gel filtration, and the like. *(Protein Engineering*, 7, 697-704, 1994).

### (7) Preparation of CDR-containing peptide

CDR-containing peptide can be prepared by a chemical synthesis method such as an Fmoc method or a tBoc method. Further, DNA coding for a CDR-containing peptide is prepared and the resulting DNA is cloned to an appropriate vector for expression whereby a CDR-containing peptide expression vector can be prepared. With regard to a vector for expression, any vector may be used so long as the DNA which codes for CDR-containing peptide can be inserted and expressed. Examples thereof are pLEX (manufactured by Invitrogen) and pAX4a+ (manufactured by Invitrogen). The expression vector is introduced into an appropriate *E. coli* and formed and accumulated in an inclusion body or periplasmic space. From the inclusion body or the periplasm, CDR-containing peptide is obtained and can be purified by an ion-exchange chromatography and a gel filtration (*Protein Engineering,* 7, 697-704, 1994).

### (8) Evaluation of activity of antibody fragments

Evaluation of activity of the purified antibody fragment can be carried out in the same manner as in the above-mentioned 1(7)·

### Best Mode for Carrying Out the Invention

The present invention is illustrated more specifically below by referring to Examples. However, these Examples do not limit the invention at all.

### (Example 1)

Examination of an effect provided by using irradiation and an anti-IGF-I monoclonal antibody in combination

Epidermoid carcinoma cell strain A431 cells (ATCC CRL-1555) were cultured in a 10-centimeter dish. When the cells were 70% confluent, a monoclonal antibody which binds to IGF-I and IGF-II and inhibits activities of IGF-I and IGF-II (hereinafter referred to as anti-IGF monoclonal antibody) KM 1468 which was produced by hybridoma FERM BP-7478 which was prepared in Reference Example 1 was added to the dish so that the final concentration became 100 ng/mL, and irradiation with X rays of 4 Gy was then conducted. At this time, cells in a dish which were not irradiated with X rays were kept as cells without X-ray irradiation. After 30 minutes from the X-ray irradiation, A431 cells were removed from the dish by trypsin treatment to recover A431 cells. Further, anti-IGF monoclonal antibody KM 1468 was added in the beginning of the culturing at a cell density of 10,000 cells/dish so that the final concentration became 100 ng/mL, and the culturing was conducted for 10 days. After the culturing, the number of colonies formed was counted. The relative value of the number of colonies are shown in Table 1 below.

**Table 1**

| Treatment method | Relative value of number of colonies |
|---|---|
| X-ray irradiation+KM 1468 | 20% or less |
| X-ray irradiation | 50% |
| KM 1468 | 100% |

In comparison to no X-ray irradiation, X-ray irradiation decreased the number of colonies to approximately 50%. The addition of anti-IGF monoclonal antibody KM 1468 decreased the number of colonies to 20% or less. In view of the foregoing, it has been confirmed that the combined use of irradiation and the anti-IGF-1 monoclonal antibody is useful for treating cancer.

### (Example 2)

Examination of an effect provided by using an agent having low-molecular weight and an anti-IGF-I monoclonal antibody in combination

An agent diluted stepwise and anti-IGF monoclonal antibody KM 1468 diluted stepwise were added to a 96-well culture plate at 50 µL/well each. Further, multiple myeloma cell line, LP-1 cells (DSMZ ACC41) were added at 100 µL/well (10,000 cells) each, and the mixture was cultured at 37°C for 3 days. After the culturing, cell proliferation reagent WST-1 (manufactured by Roche) was added at 20 µL/well. The mixture was cultured at 37°C for 2 to 3 hours, and OD450 was measured with microplate reader M-SPmax 250 (manufactured by Molecular Devices). The growth inhibitory concentration of the agent was expressed as a relative value when OD450 in which cells were solely added was regarded as 100%. 5 to 70% growth inhibitory concentration (IC₅ to IC₇₀) on which the agent or the antibody was added, and IC₅₀ and IC₇₀ on which the agent and the antibody were used in combination were calculated, respectively, and the effect provided by the combined use was analyzed using an isoborogram method (International Journal of Radiation Oncology, Biology, Physics, 5, 85-91, 1979). Melphalan, nimustin, doxorubicin, mitoxantrone, vinorelbin, etoposide, paclitaxel, dexamethasone, 5-fluorouracil, methotrexate, gemcitabine, cisplatin, thalidomide, 7-ethyl-10-hydroxycamptothecine (active substance of irinotecan; hereinafter abbreviated as SN-38; Cancer Research, 50, 1715-1721, 1990), rapamycin, radicicol, 17-allylamino-17-demethoxygeldanamycin (hereinafter abbreviated as 17AAG; Cancer Chemotherapy & Pharmacology, 42, 273-279,1998),UCN-01 (Journal of Antibiotics, 40, 1782-1784, 1987), PD173074 (EMBO Journal, 17, 5896-5904, 1998), ZD6474 (Cancer Research, 62, 4645-4655, 2002), N-(3-amino-propyl)-N-[1-(3-benzyl-7-chloro-4-oxo-3,4-dihydro-quinazolin-2-yl)-2-methyl-propyl]-4-methyl-benzamide (GlaxoSmithKline, WO 01/98278, WO 03/070701), N⁴ Quinolin-3-yl-N²-(3,4,5-trimethoxyphenyl)pyrimidine-2,4-diamine (Amgen, WO 03/018021) were used respectively as the agent used in combination.

It has been confirmed that the combined use of the agent having low-molecular weight and the anti-IGF monoclonal antibody is effective for treating cancer.

### (Example 3)

Examination of an administration method in the combined use of a agent having low-molecular weight and an anti-IGF-1 monoclonal antibody

Multiple myeloma cell line, LP-1 cells (DSMZ ACC41) were seeded in a 96-well culture plate in an amount of 100 µL/well (10,000 cells). Doxorubicin and anti-IGF monoclonal antibody KM 1468 were added, and the mixture was cultured at 37°C for 3 days. The addition of doxorubicin and KM 1468 to the medium was conducted by the following three methods.
Method 1: Cells were cultured in 150 µL of a medium in which doxorubicin was solely added on day 1, and in 200 µL of a medium in which doxorubicin and KM 1468 were added on days 2 and 3.
Method 2: Cells were cultured in 150 µL of a medium in which KM 1468 were solely added on day 1, and in 200 µL of a medium in which doxorubicin and KM 1468 were added on days 2 and 3.
Method 3: Cells were cultured in a medium comprising doxorubicin and KM 1468 (an amount of the medium is 150 µL on day 1 and 200 µL each on days 2 and 3) for three days.

In all of the foregoing methods, doxorubicin and KM 1468 were added so that the final concentrations of doxorubicin and KM 1468 became 1 µmol/L and 1 µg/mL, respectively.

After the culturing, cell proliferation reagent WST-1 (manufactured by Roche) was added in an amount of 20 µL/well, and the mixture was cultured at 37°C for from 2 to 3 hours. OD450 was measured with microplate reader M-SPmax 250 (manufactured by Molecular Devices). The number of viable cells after the treatment with the agent was expressed as a relative value when OD450 in which cells were solely added was regarded as 100%. The results are shown in Table 2 below.

**Table 2**

| Treatment method | Relative value of cell number |
|---|---|
| Doxorubicin only | 40% |
| KM 1468 only | 49% |
| Method 1 | 21% |
| | |

| Treatment method | Relative value of cell number |
|---|---|
| Doxorubicin only | 57% |
| KM 1468 only | 29% |
| Method 2 | 19% |
| | |

| Treatment method | Relative value of cell number |
|---|---|
| Doxorubicin only | 44% |
| KM 1468 only | 26% |
| Method 3 | 11% |

In all of the administration methods, the combined use has strongly inhibited the proliferation of cells in comparison to the single use. Accordingly, it has been confirmed that all of the administration methods of the agent having low-molecular weight and the anti-IGF monoclonal antibody are effective for treating cancer.

### (Reference Example 1) Preparation of anti-hIGF monoclonal antibody

### (1) Immunization of animal and preparation of antibody-producing cell

A recombinant hIGF-I (manufactured by R & D) was made into a complex with a methylated BSA (manufactured by Sigma) for the purpose of increasing its immunogenicity, and use as the immunogen. Thus, methylated BSA dissolved in redistilled water was mixed at 4°C so as to make methylated BSA:hIGF = 1:4 (ratio by weight) and stirred for 10 seconds in a vortex mixer. After that, it was mixed with complete Freund's adjuvant or incomplete Freund's adjuvant using a syringe equipped with a connecting needle at a ratio by volume of 1:1 to give an immunogen (hereinafter, referred to as methylated BSA-hIGF-I).

The methylated BSA-hIGF-I (equivalent to 100 µg of hIGF-I) prepared as above using a complete Freund's adjuvant was administered to a female SD rat of 5-weeks old and, from two weeks thereafter, an immunogen which was similarly prepared using an incomplete Freund's adjuvant was administered once a week for 4 times in total.

Blood was collected from venous plexus of the fundus of the eye, antibody titer in its serum was checked by a binding ELISA shown in Reference Example 1(4) and spleen was excised from a rat showing a sufficient antibody titer after 3 days from the final immunization.

After the spleen was cut into pieces in an MEM medium (manufactured by Nissui Seiyaku), loosened by tweezers and centrifuged (at 1,200 rpm for 5 minutes), the supernatant was discarded, the resulting precipitate was treated with a Tris-ammonium chloride buffer (pH 7.65) for 1 to 2 minutes to eliminate erythrocytes, and the remainder was washed with MEM for 3 times to be used for cell fusion.

### (2) Preparation of mouse myeloma cells

An 8-azaguanine-resistant mouse myeloma cell line P3-U1 was incubated in a common medium and not less than 2 × 10⁷ cells were secured upon cell fusion to be used as a parent cell for cell fusion.

### (3) Preparation of hybridoma

The rat spleen cell prepared in Reference Example 1 (1) and the myeloma cell prepared in (2) were mixed so as to make their ratio 10:1 followed by centrifuging (at 1,200 rpm for 5 minutes), the supernatant was discarded, 0.2 to 1.0 mL of a fusion medium (a mixture of 2 g of PEG 1000, 2 mL of MEM and 0.7 mL of dimethyl sulfoxide) per 10² rat spleen cell was added to the precipitated cell with stirring at 37°C, 1 to 2 mL of MEM was added for several times every 1 to 2 minutes and MEM was further added thereto so that the total volume was made 50 mL. After centrifugation (at 900 rpm for 5 minutes), the supernatant was discarded and the resulting cell were gently loosened and suspended in 100 mL of HAT medium.

The suspension was dispensed in a 96-well plate for incubation in an amount of 100 µL/well and incubated in a 5% CO₂ incubator for 10 to 14 days at 37°C. The culture supernatant was subjected to a binding ELISA shown in Reference Example 1(4) to select wells which reacted with methylated BSA-hIGF-I and did not react with methylated BSA-BSA which is a negative control [a complex prepared by the same reaction as in the above Referential Example 1(1) using BSA], and anti-hIGF-I rat monoclonal antibody-producing hybridoma were established by carrying out single cell cloning twice by changing the medium to HT medium and the normal medium.

As a result, 6 hybridoma clones of KM 1468, KM 1469, KM 1470, KM 1471, KM 1472 and KM 1473 having reactivities shown in Fig. 11 were obtained. When subclass of the antibody produced by each hybridoma was examined by an ELISA using a subclass typing kit, all of the subclasses were IgG2b.

### (4) Selection of monoclonal antibody (combined ELISA)

As to an antigen to be immobilized to an ELISA plate, the methylated BSA-hIGF-I prepared in Reference Example 1(1) was used while, as to a negative control, methylated BSA-BSA was used. The above antigen in 10 µg/mL in terms of concentration of hIGF-1 or BSA was dispensed in a 96-well ELISA plate (manufactured by Greiner) in an amount of 50 µL/well and allowed to stand over night at 4°C for immobilization. After washing with PBS, PBS containing 1% of BSA (hereinafter, referred to as BSA-PBS) was added in an amount of 100 µL/well and reacted at room temperature for 1 hour to block the remaining active group. The BSA-PBS was discarded and then rat antiserum to be immunized, culture supernatant of hybridoma which produces anti-hIGF-I monoclonal antibody or purified anti-hIGF-I monoclonal antibody was dispensed in an amount of 50 µL/well and reacted at room temperature for 2 hours. After the reaction, each well was washed with PBS containing 0.05% of Tween 20 (hereinafter, referred to as Tween-PBS) and 50 f.tL/well of peroxidase-labeled rabbit anti-rat Ig antibody diluted to 4,000-fold (manufactured by Dako) was added as a secondary antibody and allow to react at room temperature for 1 hour. After the reaction, it was washed with Tween-PBS, 50 µL/well of ABTS substrate solution [a solution prepared by dissolving 0.55 g of ammonium 2,2'-azino-bis(3-ethylbenzothiazoline-6-sulfonate) in 1L of 0.1 M citrate buffer (pH 4.2) followed by adding 1 µL/ml of an aqueous solution of hydrogen peroxide immediately before use] was then added thereto to effect color development and absorbance at 415 nm (hereinafter, referred to as OD415) was measured using a plate reader Emax (manufactured by Molecular Devices).

### (5) Purification of monoclonal antibody

The hybridoma clone prepared in Reference Example 1(3) was intraperitoneally injected in an amount of 5 to 20 × 10⁶ cells/mouse into pristane-treated 8-weeks old female Balb/c nude mice. After 10 to 21 days, ascites was collected (1 to 8 mL/mouse) from the mice where the hybridoma turned ascites cancer and centrifuged (at 3,000 rpm for 5 minutes) to remove solids. After that, IgG fraction was purified by a caprylic acid precipitation method (Antibodies, A Laboratory Manual, Cold Spring Harbor Laboratory, 1988) to give a purified monoclonal antibody.

### (Reference Example 2) Examination of reactivity of anti-hIGF monoclonal antibody

### (1) Reactivity of hIGF-1 to natural three-dimensional structure

Reactivity of the anti-hIGF monoclonal antibody selected in Referentce Example 1(3) to hIGF-I maintaining natural three-dimensional structure in a liquid phase system was examined by the following competitive ELISA.

A plate where the methylated BSA-hIGF-I prepared in Reference Example 1(1) was immobilized as shown in Reference Example 1(4) was prepared, hIGF-I which was diluted in 5-fold serial dilutions from 20 µg/mL was dispensed in an amount of 50 µL/well, then a solution where the purified antibody of the anti-hIGF monoclonal antibody was diluted (KM 1468: 6.0 µg/ML, KM 1470: 1.0 µg/mL, KM 1471: 0.16 µg/mL, KM 1472: 7.0 µg/mL, KM 1473: 1.2 µg/mL) was dispensed in an amount of 50 µL/well followed by mixing and the mixture was allowed to react at room temperature for 2 hours. After the reaction, it was washed with Tween-PBS and then 50 µL/well of peroxidase-labeled rabbit anti-rat Ig antibody (manufactured by Dako) diluted to 4,000-fold was added followed by reacting at room temperature for 1 hour. After the reaction, it was washed with Tween-PBS, 50 µL/well of an ABTS substrate solution [a solution prepared by dissolving 0.55 g of ammonium 2,2'-azino-bis(3-ethylbenzothiazoline-6-sulfonate) in 1L of 0.1 M citrate buffer (pH 4.2) followed by adding 1 µL/ml of an aqueous solution of hydrogen peroxide immediately before use] was added thereto to effect color development and OD415 was measured using a plate reader Emax (manufactured by Molecular Devices).

As shown in Fig. 2, all of the six anti-hIGF monoclonal antibodies of the present invention showed reactivity to a natural three-dimensional structure of hIGF-I. In addition, when KM 1468 showing the highest sensitivity in the present system was used, hIGF-1 having a natural three-dimensional structure contained in the liquid phase system can be detected up to a concentration of 16 ng/mL.

### (2) Reactivity of the anti-hIGF monoclonal antibody to hIGF family

Reactivity of the purified anti-hIGF monoclonal antibody KM 1468 (hereinafter, referred to as antibody MK 1468) to hIGF was examined. Fig. 3 shows the result of the examinnation on the reactivity of the antibody KM 1468 and sm1.2 (manufactured by Upstate Biotechnology) which is a commercially available anti-hIGF-I antibody to hIGF-I by binding ELISA shown in Reference Example 1(4) (in which antibody concentration was diluted in a 3-fold serial dilutions from 30 µg/mL) . In the case of sm1.2 however, a peroxidase-labeled rabbit anti-mouse Ig antibody (manufactured by Dako) diluted to 2,000-fold was used as a secondary antibody. Although any of the antibodies showed a hIGF-I-binding activity dependent upon concentration of the antibody as shown in Fig. 3, the activity was higher in the case of the antibody KM 1468.

Then an inhibitory activity by hIGF-I (manufactured by Pepro Tech EC), hIGF-II (manufactured by Pepro Tech EC), human insulin (manufactured by Wako Pure Chemical) and mIGF-I (manufactured by Pepro Tech EC) in a binding of each antibody to hIGF-I was examined by the following competitive ELISA.

As shown in Reference Example 1(4), a plate where antigen was immobilized was prepared, each antibody diluted to 4.0 µg/mL was dispensed in an amount of 50 µL/well, then hIGF-I or hIGF-II diluted in a 3-fold dilution step from 20 µg/mL or human insulin or mIGF-I diluted in a 5-fold serial dilutions from 10 µg/mL was dispensed in an amount of 50 µL/well and they were mixed and reacted at room temperature for 1 hour. After the reaction, it was washed with Tween-PBS and, in the case of KM 1468, a peroxidase-labeled rabbit anti-rat Ig antibody (manufactured by Dako) diluted to 4,000-fold or, in the case of sm1.2, a peroxidase-labeled rabbit anti-rat Ig antibody (manufactured by Dako) diluted to 2,000-fold was added in an amount of 50 µL/well followed by reacting at room temperature for 1 hour. After the reaction, it was washed with Tween-PBS, 50 µL/well of ABTS substrate solution [a solution prepared by dissolving 0.55 g of ammonium 2,2'-azino-bis(3-ethylbenzothiazoline-6-sulfonate) in 1L of 0.1 M citrate buffer (pH 4.2) following by adding with 1 µL/ml of an aqueous solution of hydrogen peroxide immediately before use] was added thereto to effect color development and OD415 was measured using a plate reader Emax (manufactured by Molecular Devices). The result was given in terms of a relative value (%) where the OD415 when only antibody was added was defined as 100. The result was shown in Fig. 4. As shown in Fig. 4, binding of the antibody KM 1468 to hIGF-I was strongly inhibited by hIGF-I (Fig. 4A) and hIGF-II (Fig. 4B) and a 50% inhibition concentration (hereinafter, referred to as IC₅₀) for the binding by hIGF-I was about 0.3 µg/mL (about 39 nM) while the IC₅₀ by HIGF-II was about 0.4 µg/mL (about 58 nM) whereby they showed nearly the same value. On the other hand, no inhibition was noted in human insulin and mIGF-I. From the above result, it has been clarified that the antibody KM 1468 reacts with both hIGF-I and hIGF-II almost the same specificity and almost the same degree. Binding of sm1.2 which is the commercially available anti-IGF-1 antibody to hIGF-I was strongly inhibited by hIGF-I (Fig. 4A) and an inhibitory activity by hIGF-II (Fig. 4B) was weak. IC₅₀ of sml.2 by hIGF-I was about 1.2 µg/mL (about 156 nM) while IC₅₀ by hIGF-II was >10 µg/mL (> 1.45 µM). On the other hand, no inhibition was noted in human insulin and mIGF-I.

### (3) Influence of anti-hIGF monoclonal antibody on hIGF-dependent cell proliferation

Influence of purified antibody KM 1468 on hIGF-dependent cell proliferation was examined. With regard to the antibody, KM 1468, sml.2 (manufactured by Upstate Biotechnology) which is a commercially available anti-hIGF-I antibody and S1F2 (manufactured by Upstate Biotechnology) which is a commercially available anti-hIGF-II antibody were used.

Human breast cancer cell line MCF 7 (ATCC HTB-22), human colorectal cancer cell line HT-29 (ATCC HTB-38) or human osteosarcoma cell line MG-63 (ATCC CRL-1427) was prepared into 0.5 to 1 × 10⁵ cells/mL using a TF/BSA medium [a medium where 10 µg/mL of human transferrin (manufactured by Gibco BRL) and 200 µg/mL of BSAwere added to D-MEM/F-12 (manufactured by Gibco BRL)] and dispensed in a 96-well plate for incubation in an amount of 100 µL/well. Subsequently, each factors of hIGF-I, hIGF-II and human insulin diluted to each concentration with the TF/BSA medium was added in amount of 50 µl/well thereto, and each of the antibodies diluted to each concentration with the TF/BSA medium was added in amount of 50 µl/well, and cultured at 37°C for 5 days in a 5% CO₂ incubator. After incubation, a cell proliferation reagent WST-1 (manufactured by Roche) was dispensed in an amount of 20 µL/well and incubated for 2.5 to 4 hours in a 5% CO₂ incubator at 37°C and then absorbance at OD450 nm (hereinafter, referred to as OD450) was measured using a plate reader Emax (manufactured by Molecular Devices).

Fig. 5A shows a proliferation curve of human breast cancer cell line MCF 7 by each factor. Further, Fig. 5B shows proliferation upon addition of each antibody in the presence of 40 ng/mL of hIGF-I, Fig. 5C shows the proliferation in the presence of 100 ng/mL of hIGF-II and Fig. 5D shows the proliferation in the presence of 100 ng/mL of human insulin. As shown in Fig. 5, KM 1468 strongly inhibits the cell proliferation by hIGF-I and hIGF-II in the same degree and its activity was higher than sml.2 which is the commercially available anti-hIGF-I antibody and than S1F2 which is a commercially available anti-hIGF-II antibody. On the other hand, none of antibodies affected the growth by human insulin. The above result clearly shows that there is a good correlation to the binding specificity of each antibody seen in the competitive ELISA of Reference Example 2(2) and that activity of hIGF-I and hIGF-II is inhibited by binding of each antibody.

Fig. 6A shows a proliferation curve of human colorectal cancer cell line HT-29 by each factor. Fig. 6B shows proliferation upon addition of each antibody in the presence of 10 ng/mL of hIGF-I, Fig. 6C shows the proliferation in the presence of 10 ng/mL of hIGF-II and Fig. 6D shows the proliferation in the presence of 20 ng/mL of human insulin.

As shown in Fig. 6, KM 1468 strongly inhibits the cell proliferation by hIGF-I and hIGF-II in the same degree and its activity was higher than sml.2 which is the commercially available anti-hIGF-I antibody as well as S1F2 which is a commercially available anti-hIGF-II antibody. On the other hand, none of antibodies affected the proliferation by human insulin. The above result clearly shows that there is a good correlation to the binding specificity seen in the competitive ELISA of Reference Example 2(2) and that activity of hIGF-1 and hIGF-II is inhibited by binding of each antibody. Further, when KM 1468 of Fig. 6B and KM 1468 and S1F2 of Fig. 6C were allowed to react, cell proliferation was suppressed as compared with the case where hIGF-I and hIGF-II were not added. From the above, it became clear that HT-29 cell are proliferated by producing hIGF-I and hIGF-II by themselves and that the proliferation effect can also be inhibited by addition of antibody.

Fig. 7A shows a proliferation curve of human osteosarcoma cell line MG-63 by each factor. Fig. 7B shows proliferation upon addition of each antibody in the presence of 20 ng/mL of hIGF-I, Fig. 7C shows the proliferation in the presence of 20 ng/mL of hIGF-II and Fig. 7D shows the proliferation in the presence of 20 ng/mL of human insulin. As shown in Fig. 7, KM 1468 strongly inhibits the cell proliferation by hIGF-I and hIGF-II in the same degree and its activity was higher than sm1.2 which is the commercially available anti-hIGF-I antibody as well as S1F2 which is a commercially available anti-hIGF-II antibody. On the other hand, none of antibodies affected the proliferation by human insulin. The above result clearly shows that there is a good correlation to the binding specificity seen in the competitive ELISA of Reference Example 2 (2) and that function of each factor is inhibited by binding of each antibody.

### (Reference Example 3) Analysis of antigen-recognizing site of anti-hIGF monoclonal antibody

### (1) Synthesis of partial peptide of hIGF-I

A partial peptide of hIGF-I was synthesized according to a method mentioned in WO 01/64754. The synthesized peptide is a peptide corresponding to 1st to 18th (SEQ ID NO: 1; hereinafter, referred to as p1-18), 14th to 30th (SEQ ID NO: 2; hereinafter, referred to as p14-30), 24th to 35th (SEQ ID NO: 3; hereinafter, referred to as p24-35), 29th to 41st (SEQ ID NO: 4; hereinafter, referred to as p29-41), 36th to 47th (SEQ ID NO: 5; hereinafter, referred to as p36-47), 41st to 56th (SEQ ID NO: 6; hereinafter, referred to as p41-56), 52nd to 70th (SEQ ID NO: 7; hereinafter, referred to as p52-70), 53rd to 61st (SEQ ID NO: 8; hereinafter, referred to as p53-61) and 61st to 70th (SEQ ID NO: 9; hereinafter, referred to as p61-70) of hIGF-I and was designed to cover the full length of hIGF-I. In the above-mentioned peptides, a sequence where Cys existing therein was substituted with Ser or Ala was synthesized. With regard to the sequence corresponding to 41st to 56th, a sequence having an inner Cys (SEQ ID NO: 10; hereinafter, referred to as p41-56C) was also synthesized.

### (2) Analysis of antigen-recognizing site of anti-hIGF monoclonal antibody

Analysis of antigen-recognizing site of anti-hIGF rat antibody KM 1468 was examined by the following competitive ELISA using various kinds of peptides synthesized in the above (1).

As shown in Reference Example 1(4), a plate where antigen was immobilized was prepared, various antibodies diluted to 4.0 µg/mL were dispensed in 50 µL/well and either alone or various combinations of various peptide solutions diluted in 3-fold serial dilutions from 50 µg/mL or hIGF-I was dispensed in 50 µL/well followed by mixing and reacting at room temperature for 1 hour. After the reaction, the above was washed with Tween-PBS, a peroxidase-labeled rabbit anti-rat Ig antibody (manufactured by Dako) diluted to 4,000-fold was added in an amount of 50 µL/well and was allowed to react at room temperature for 1 hour. After the reaction, it was washed with Tween-PBS, an ABTS substrate solution [a solution prepared by dissolving 0.55 g of ammonium 2,2'-azino-bis(3-ethylbenzothiazoline-6-sulfonate) in 1L of 0.1M citrate buffer (pH 4.2) followed by adding 1 µL/mL of aqueous hydrogen peroxide solution thereto immediately before use] was added thereto in an amount of 50 µL/well to effect color development and OD415 was measured using a plate reader Emax (manufactured by Molecular Devices). The result is shown in terms of a relative value (%) where the OD415 when only antibody was added was defined as 100. The result is shown in Fig. 8. As shown in Fig. 8, binding of KM 1468 to hIGF-I was inhibited by hIGF-I depending on concentration but, in the cases of various peptides, no inhibitory activity was noted regardless of sole or combined use thereof. The above result strongly suggests that KM 1468 is not a mere amino acid primary sequence of hIGF-I but recognizes a three-dimensional structure of hIGF-I.

All of the publications, patents and patent applications cited in the present specification are incorporated as such into the present specification by reference.

### Industrial Applicability

The medicament for treating cancer of the invention can enhance the effect for treating cancer by combining the substance inhibiting the activities of insulin-like growth factor-I (IGF-I) and insulin-like growth factor-II (IGF-II) with irradiation or the substance having the antitumor activity in comparison to single administration of each of these substances. According to this combination therapy, in comparison to the single administration, the effects such as the dose of the agent can be reduced, the substance used in combination can be selected according to conditions (a type of a cancer and a degree of seriousness of a cancer) of patients, a therapeutic period is kept by selecting the substances used in combination and different in activity, are advantageous.

## Claims

1. A medicament for treating cancer which comprises a substance inhibiting activities of insulin-like growth factor-I (IGF-I) and insulin-like growth factor-II (IGF-II) and which is administered in combination with irradiation.

2. The medicament for treating cancer according to claim 1, wherein the irradiation is conducted once or plural times at the time of administrating the medicament for treating cancer, or before or after the administration.

3. A medicament for treating cancer which comprises a combination of a substance inhibiting activities of insulin-like growth factor-I (IGF-I) and insulin-like growth factor-II (IGF-II) and a substance having an antitumor activity.

4. The medicament for treating cancer according to claim 3, wherein the substance inhibiting activities of insulin-like growth factor-I (IGF-I) and insulin-like growth factor-II (IGF-II) and the substance having an antitumor activity are administered simultaneously or consecutively.

5. The medicament for treating cancer according to any one of claims 1 to 4, wherein the substance inhibiting activities of insulin-like growth factor-I (IGF-I) and insulin-like growth factor-II (IGF-II) is selected from the group consisting of the following (a) to (d),
(a) an antibody or an antibody fragment which specifically binds to IGF-I and IGF-II to inhibit the activities of IGF-I and IGF-II,
(b) a composition comprising an antibody or an antibody fragment which specifically binds to IGF-I to inhibit the activity of IGF-I and an antibody or an antibody fragment which specifically binds to IGF-II to inhibit the activity of IGF-II,
(c) a component wherein an antibody or an antibody fragment which specifically binds to IGF-I to inhibit the activity of IGF-I and an antibody or an antibody fragment which specifically binds to IGF-II to inhibit the activity of IGF-II are combined, and
(d) a complex of an antibody or an antibody fragment which specifically binds to IGF-I to inhibit the activity of IGF-I and an antibody or an antibody fragment which specifically binds to IGF-II to inhibit the activity of IGF-II.

6. The medicament for treating cancer according to claim 5, wherein the antibody is a monoclonal antibody.

7. The medicament for treating cancer according to claim 6, wherein the monoclonal antibody is a monoclonal antibody which binds to an epitope to which a monoclonal antibody produced from hybridoma KM 1468 (FERM BP-7978) binds.

8. The medicament for treating cancer according to any one of claims 5 to 7, wherein the antibody fragment is an antibody fragment selected from the group consisting of Fab, Fab', F(ab')₂, a single chain antibody (scFv), a dimeric variable region (Diabody), a disulfide stabilized variable region (dsFv) and a CDR-containing peptide.

9. The medicament according to claims 1 to 8, wherein the substance having the antitumor activity is a protein or a agent having low-molecular weight.

10. The medicament according to claim 9, wherein the protein is an antibody or a cytokine.

11. The medicament according to claim 9, wherein the agent having low-molecular weight is an agent selected from the group consisting of a DNA alkylating agent, a DNA synthesis inhibitor, a platinum preparation-type DNA crosslinking agent, a metabolic antagonist, a topoisomerase I inhibitor, a topoisomerase II inhibitor, a tubulin acting agent, a hormone antagonist, an aromatase inhibitor, an immunomodulator, an immunosuppressant, a steroidal antiinflammatory agent, a non-steroidal antiinflammatory agent, an antihistaminic agent, a differentiation inducer, a proteasome inhibitor, a tyrosine kinase inhibitor, an adenosine deaminase inhibitor, an angiogenesis inhibitor, a histone deacetylase inhibitor, a matrix metalloproteinase inhibitor, a farnesyl transferase inhibitor, a bisphosphonate preparation, an Hsp90 inhibitor, a kinesin Eg5 inhibitor, a serine threonine kinase inhibitor and derivatives of these compounds.

12. A method for treating cancer which comprises administering to a mammal an effective amount of a substance inhibiting activities of insulin-like growth factor-I (IGF-I) and insulin-like growth factor-II (IGF-II) in combination with irradiation.

13. The method for treating cancer according to claim 12, wherein the irradiation is conducted once or plural times at the time of administering a medicament for treating cancer, or before or after the administration.

14. A method for treating cancer which comprises administering to a mammal an effective amount of a substance inhibiting activities of insulin-like growth factor-I (IGF-I) and insulin-like growth factor-II (IGF-II) and an effective amount of a substance having an antitumor activity in combination.

15. The method for treating cancer according to claim 14, wherein the effective amount of the substance inhibiting the activities of insulin-like growth factor-I (IGF-I) and insulin-like growth factor-II (IGF-II) and the effective amount of the substance having the antitumor activity are administered simultaneously or successively.
